# EUROPEAN PATENT APPLICATION

(11) **EP 3 865 468 A1**
(43) Date of publication of application: **18.08.2021**
(21) Application number: 19870770.5
(22) Date of filing: 08.10.2019
(51) Int. Cl.: C07C 17/358, C07C 17/35, C07C 21/22, C07C 23/06, C07B 61/00

(54) **PROCESS FOR PRODUCING PERFLUOROALKYNE COMPOUND**

(30) Priority: 09.10.2018 JP 2018191002; 23.10.2018 JP 2018199158; 16.11.2018 JP 2018215731
(71) Applicant: DAIKIN INDUSTRIES, LTD., Osaka 530-8323 (JP)
(72) Inventor: ETOU, Yuusuke, Osaka-shi, Osaka 530-8323 (JP); NAKAMURA, Shingo, Osaka-shi, Osaka 530-8323 (JP)
(74) Representative: Hoffmann Eitle
(86) International application number: PCT/JP2019/039725
(87) International publication number: WO 2020/075728

(57) **Abstract**

Disclosed is a method for producing a perfluoroalkyne compound,
the method using a catalyst comprising any one of the following catalysts (a) to (e):
(a) a catalyst containing a specific element,
(b) a catalyst containing at least two specific elements,
(c) a fluorinated specific metal oxide having a pore volume within a predetermined range,
(d) a metal oxide fluorinated by a specific compound, and
(e) a catalyst obtained by fluorinating a specific metal oxide having a pore volume within a predetermined range; or
the method using a catalyst comprising a specific element, and comprising brining the catalyst into contact with the perfluoroalkadiene compound for 30 seconds or less; or
the method performing at least a part of the reaction of the perfluoroalkadiene compound from the start to the end of the reaction under conditions in which the reaction system has a water content within a specific range. The production method can produce a perfluoroalkyne compound with a high selectivity at a high conversion rate of the reaction, while being less likely to produce a fluoroalkene compound as a by-product. This production method can also reduce catalyst degradation.

## Description

### Technical Field

This disclosure relates to a method for producing a perfluoroalkyne compound.

### Background Art

Perfluoroalkyne compounds are compounds that are expected to be useful as a dry etching gas for semiconductors; and also in various applications such as refrigerants, foaming agents, and heat transfer media. Perfluoroalkyne compounds are compounds that have one carbon-carbon triple bond.

As a method for producing such a perfluoroalkyne compound, an isomerization reaction of hexafluorobutadiene is known. For example, Patent Literature (PTL) 1 discloses isomerizing hexafluorobutadiene to obtain hexafluorobutyne using halogenated alumina. Patent Literature (PTL) 2 discloses using sodium fluoride or a mixture containing sodium fluoride as an isomerization reaction catalyst to obtain hexafluorobutyne from hexafluorocyclobutene.

### Citation List

### Patent Literature

PTL 1: JP2012-001448A
PTL 2: JP2014-058488A

### Summary of Invention

### Technical Problem

An object of the present disclosure is to provide a method that can produce a perfluoroalkyne compound with a high selectivity at a high conversion rate of the reaction, and that is less likely to produce a fluoroalkene compound as a by-product. Another object of the present disclosure to provide a method for producing a perfluoroalkyne compound, the method being capable of reducing catalyst degradation.

### Solution to Problem

The present disclosure includes the following.
Item 1. A method for producing a perfluoroalkyne compound, the method comprising reacting a perfluoroalkadiene compound in the presence of a catalyst to obtain a perfluoroalkyne compound, and satisfying one of the following (A) to (G):
   (A) the catalyst comprises at least one catalyst selected from the group consisting of catalysts containing a transition metal element, and catalysts containing at least two elements that belong to Groups 3 to 14 of the periodic table of elements;
   (B) the catalyst comprises a catalyst containing at least one element that belongs to Groups 3 to 14 of the periodic table, and the contact time between the catalyst and the perfluoroalkadiene compound is 30 seconds or less;
   (C) the catalyst comprises at least one catalyst selected from the group consisting of fluorinated chromium oxide having a pore volume of 0.08 mL/g or more, fluorinated alumina having a pore volume of 0.35 mL/g or more, and fluorinated silica alumina having a pore volume of 0.50 mL/g or more;
   (D) the catalyst comprises a fluorinated metal oxide having a pore volume of 0.35 mL/g or more;
   (E) the catalyst comprises a metal oxide fluorinated by reacting a metal oxide with at least one compound selected from the group consisting of hydrofluorocarbon, hydrochlorofluorocarbon, and chlorofluorocarbon;
   (F) the catalyst comprises one or more catalysts obtained by fluorinating at least one metal oxide selected from the group consisting of chromium oxide having a pore volume of 0.10 mL/g or more, alumina having a pore volume of 0.45 mL/g or more, and silica alumina having a pore volume of 0.50 mL/g or more; and
   (G) at least a part of the reaction of the perfluoroalkadiene compound from the start to the end of the reaction is performed under conditions in which the water content of the reaction system is 30 ppm by mass or less, based on the mass of the perfluoroalkadiene compound (defined as 100 mass%).
Item 2. The production method according to Item 1, wherein the perfluoroalkyne compound is represented by formula (1):

   CR¹₂R²-C≡C-CR³R⁴₂ (1)

   (wherein R¹ to R⁴ are the same or different and represent a fluorine atom or a perfluoroalkyl group).
Item 3. The production method according to Item 1 or 2, wherein the perfluoroalkadiene compound is represented by formula (2):

   CR¹₂=CR²-CR³=CR⁴₂ (2)

   (wherein R¹ to R⁴ are the same or different and represent a fluorine atom or a perfluoroalkyl group).
Item 4. The production method according to any one of Items 1 to 3, wherein the method satisfies the (A) or (B), and the catalyst is at least one catalyst selected from the group consisting of: catalysts containing at least one transition metal element that belongs to Groups 4 to 6 of the periodic table; and catalysts containing at least two elements that belong to Groups 4 to 6 and Groups 13 to 14 of the periodic table.
Item 5. The production method according to any one of Items 1 to 4, wherein the method satisfies the (A) or (B), and the catalyst is at least one catalyst selected from the group consisting of optionally fluorinated chromium oxide catalysts, optionally fluorinated titanium oxide catalysts, optionally fluorinated zirconia catalysts, and optionally fluorinated silica-alumina catalysts.
Item 6. The production method according to any one of Items 1 to 3, wherein the method satisfies the (D), and the metal of the fluorinated metal oxide includes at least one element that belongs to Groups 3 to 14 of the periodic table.
Item 7. The production method according to any one of Items 1 to 3, wherein the method satisfies the (E) and the metal oxide before fluorination has a pore volume of 0.45 mL/g or more.
Item 8. The production method according to Item 1, 2, 3, or 7, wherein the method satisfies the (E) and the metal of the metal oxide before fluorination includes at least one element that belongs to Groups 3 to 14 of the periodic table.
Item 9. The production method according to any one of Items 1 to 3, wherein the method satisfies the (G) and the catalyst contains at least one element that belongs to Groups 3 to 14 of the periodic table.
Item 10. The production method according to any one of Items 1 to 9, wherein the reaction of the perfluoroalkadiene compound is performed in a gas phase.
Item 11. The production method according to any one of Items 1 to 10, wherein the reaction of the perfluoroalkadiene compound is performed at 170°C or more.
Item 12. The production method according to any one of Items 1 to 11, wherein the reaction of the perfluoroalkadiene compound produces a perfluorocycloalkene compound in addition to the perfluoroalkyne compound.
Item 13. The production method according to Item 12, wherein the perfluorocycloalkene compound is represented by formula (3): 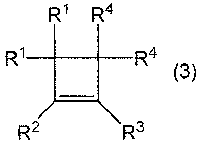 (wherein R¹ to R⁴ are as defined above) .
Item 14. A method for producing a perfluoroalkyne compound, the method comprising obtaining the perfluoroalkadiene compound using as a substrate the perfluorocycloalkene compound obtained as a by-product by the production method of Item 12 or 13.
Item 15. The production method according to Item 14, wherein the perfluoroalkyne compound is represented by formula (1):

   CR¹₂R²-C≡C-CR³R⁴₂ (1)

   (wherein R¹ to R⁴ are the same or different and represent a fluorine atom or a perfluoroalkyl group).
Item 16. A composition comprising a perfluoroalkyne compound and a perfluorocycloalkene compound,
   the perfluoroalkyne compound being represented by formula (1):

   CR¹₂R²-C≡C-CR³R⁴₂ (1)

   (wherein R¹ to R⁴ are the same or different and represent a fluorine atom or a perfluoroalkyl group),
   the perfluorocycloalkene compound being represented by formula (3) : 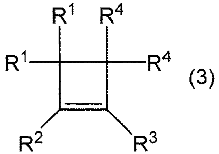 (wherein R¹ to R⁴ are as defined above),
   the composition containing the perfluoroalkyne compound represented by formula (1) in an amount of 40 to 99.999 mol%, based on the total amount of the composition defined as 100 mol%.
Item 17. The composition according to Item 16, which is for use as an etching gas or a building block for organic synthesis.
Item 18. A catalyst for use in reacting a perfluoroalkadiene compound to obtain a perfluoroalkyne compound,
   the catalyst satisfying the following (C) or (D):
   (C) the catalyst contains at least one member selected from the group consisting of fluorinated chromium oxide having a pore volume of 0.08 mL/g or more, fluorinated alumina having a pore volume of 0.35 mL/g or more, and fluorinated silica alumina having a pore volume of 0.50 mL/g or more;
   (D) the catalyst contains a fluorinated metal oxide having a pore volume of 0.35 mL/g or more.
Item 19. The catalyst according to Item 18, wherein the catalyst satisfies the (D) and the metal of the fluorinated metal oxide includes at least one element that belongs to Groups 3 to 14 of the periodic table.
Item 20. A method for producing a catalyst for use in reacting a perfluoroalkadiene compound to obtain a perfluoroalkyne compound, the method comprising the following step (E) or (F):
   (E) reacting a metal oxide with at least one compound selected from the group consisting of hydrofluorocarbon, hydrochlorofluorocarbon, and chlorofluorocarbon to thereby fluorinate the metal oxide;
   (F) fluorinating at least one metal oxide selected from the group consisting of chromium oxide having a pore volume of 0.10 mL/g or more, alumina having a pore volume of 0.45 mL/g or more, and silica-alumina having a pore volume of 0.50 mL/g or more.
Item 21. The production method according to Item 20, wherein the method satisfies the (E) and the metal oxide before fluorination has a pore volume of 0.45 mL/g or more.
Item 22. The production method according to Item 20 or 21, wherein the method satisfies the (E) and the metal of the metal oxide before fluorination includes at least one element that belongs to Groups 3 to 14 of the periodic table.

### Advantageous Effects of Invention

According to the present disclosure, there can be provided a production method that can produce a perfluoroalkyne compound with a high selectivity at a high conversion rate of the reaction, and that is less likely to produce a fluoroalkene compound as a by-product. According to the present disclosure, there can also be provided a method for producing a perfluoroalkyne compound, the method being capable of reducing catalyst degradation.

### Brief Description of Drawings

Fig. 1: Relationship between the reaction time and the conversion rate when a fluorinated alumina catalyst was used.
Fig. 2: Relationship between the pore volume before fluorination and the degradation rate when a fluorinated alumina catalyst was used.
Fig. 3: Relationship between the pore volume after fluorination and the degradation rate when a fluorinated alumina catalyst was used.

### Description of Embodiments

In the present specification, the terms "comprise," "contain," and "include" encompass the concepts of "comprising," "consisting essentially of," and "consisting of." In the present specification, a numerical range indicated by "A to B" means A or more and B or less.

### 1. Catalyst and Catalyst Production Method

The catalyst according to the present disclosure is a catalyst for use in reacting a perfluoroalkadiene compound to obtain a perfluoroalkyne compound, the catalyst satisfying the following (C) or (D):
(C) the catalyst comprises at least one member selected from the group consisting of fluorinated chromium oxide having a pore volume of 0.08 mL/g or more, fluorinated alumina having a pore volume of 0.35 mL/g or more, and fluorinated silica alumina having a pore volume of 0.50 mL/g or more;
(D) the catalyst comprises a fluorinated metal oxide having a pore volume of 0.35 mL/g or more.

### [1-1] Catalyst and Catalyst Production Method (No. 1)

In the present disclosure, catalyst 1 for use in reacting (isomerizing) a perfluoroalkadiene compound to obtain a perfluoroalkyne compound (which may be hereinafter referred to as "isomerization reaction catalyst 1") contains at least one member selected from the group consisting of fluorinated chromium oxide having a pore volume of 0.08 mL/g or more, fluorinated alumina having a pore volume of 0.35 mL/g or more, and fluorinated silica-alumina having a pore volume of 0.50 mL/g or more. This catalyst satisfies the above requirement (C). Conventionally used isomerization reaction catalysts are not optimized in pore volume. It is unclear what catalyst pore volume is conventionally used. In the present disclosure, fluorinated metal oxides having specific pore volumes are used to thereby increase the conversion rate of the reaction, and reduce catalyst degradation; accordingly, even when the isomerization reaction is performed over a long period of time, catalyst degradation can be inhibited. Therefore, the use of the catalyst of the present disclosure can reduce replacement frequency of the catalyst, and is thus economical.

The chromium oxide catalyst is not particularly limited. When chromium oxide is expressed as CrOₘ, 1<m<3 is preferable, 1.2<m<2 is more preferable, and 1.3<m<1.8 is even more preferable. When chromium oxide is expressed as CrOₘ·nH₂O, chromium oxide may be hydrated to achieve an n value of 3 or less, and particularly 1 to 1.5.

The following is an example of the method for synthesizing a chromium oxide catalyst.

First, an aqueous solution of a chromium salt (chromium nitrate, chromium chloride, chromium alum, chromium sulfate, etc.) is mixed with an aqueous ammonia to thereby obtain a precipitate of chromium hydroxide. The precipitation reaction rate at this time can control the physical properties of chromium hydroxide. A fast reaction rate is preferable. The reaction rate depends on the reaction solution temperature, the ammonia water mixing method (mixing rate), the stirring state, and the like.

This precipitate can be filtered and washed, and then dried. The drying can be performed, for example, in air at 70 to 200°C for 1 to 100 hours. The catalyst at this stage may be referred to as the chromium hydroxide state. Subsequently, the catalyst can be crushed. In view of the pellet strength, catalyst activity, etc., the precipitation reaction rate is preferably adjusted so that the crushed powder (e.g., powder having a particle size of 1000 µm or less, in particular, a particle size of 46 to 1000 µm, in a proportion of 95%) has a particle density of 0.6 to 1.1 g/ml, and preferably 0.6 to 1.0 g/ml. The powder preferably has a specific surface area (specific surface area) determined by the BET method of 100 m²/g or more, more preferably 120 m²/g or more, for example, at 200°C under degassing conditions for 80 minutes. The upper limit of the specific surface area is, for example, about 220 m²/g.

If necessary, graphite can be mixed in an amount of 3 wt.% or less into the chromium hydroxide powder, and pelletized by a tableting machine. The size and strength of the pellet can be adjusted as appropriate.

The formed catalyst can be calcined in an inert atmosphere, for example, in a nitrogen stream, to give amorphous chromium oxide. The calcination temperature is preferably 360°C or higher. In view of inhibiting crystallization, the calcination temperature is preferably 380 to 460°C. The calcination time can be, for example, 1 to 5 hours.

In view of the catalyst activity, the calcined catalyst preferably has a specific surface area of 170 m²/g or more, more preferably 180 m²/g or more, and even more preferably 200 m²/g or more. The upper limit of the specific surface area is usually preferably about 240 m²/g, and more preferably about 220 m²/g.

Examples of the alumina catalyst include α-alumina, activated alumina, and the like. Examples of the activated alumina include ρ-alumina, χ-alumina, κ-alumina, η-alumina, pseudo-γ-alumina, γ-alumina, α-alumina, θ-alumina, and the like.

Silica-alumina catalysts can also be used as composite oxide. Silica-alumina catalysts are composite oxide catalysts containing silica (SiO₂) and alumina (Al₂O₃). For example, catalysts having a silica content of 20 to 90 mass%, particularly 50 to 80 mass%, based on the total amount of silica and alumina defined as 100 mass%, can be used.

In the present disclosure, a metal oxide catalyst such as those described above is fluorinated to obtain a fluorinated metal oxide catalyst. The fluorinated metal oxide catalyst has strong activity, and the fluorination also easily reduces catalyst degradation; accordingly, even when the isomerization reaction is performed over a long period of time, catalyst degradation can be inhibited by adjustment of the pore volume. Therefore, the metal oxide catalyst is used in the form of a fluorinated metal oxide catalyst. The method for fluorinating the metal oxide catalyst is described later.

The catalyst of the present disclosure is configured to have a large pore volume because it reduces catalyst degradation and can inhibit catalyst degradation even when the isomerization reaction is performed over a long period of time. Specifically, the pore volume of each metal oxide catalyst is such that fluorinated chromium oxide has a pore volume of 0.05 mL/g or more (particularly 0.075 to 1.5 mL/g), fluorinated alumina has a pore volume of 0.35 mL/g or more (particularly 0.40 to 2.0 mL/g), and fluorinated silica-alumina has a pore volume of 0.50 ml/g or more (particularly 0.55 to 2.0 mL/g). When the pore volume is smaller than this range, the pores where active sites of the catalyst are present are covered with carbon, which is a by-product of the reaction, or the adhered carbon impairs gas diffusion in the pores, thus reducing the activity of the catalyst, i.e., being likely to cause catalyst degradation. On the other hand, an excessively large pore volume makes the catalyst production method complicated, and increases production costs.

Such a catalyst according to the present disclosure has an increased pore volume, as described above. The pore volume can be adjusted by the degree of fluorination of the catalyst; that is, the fluorine atom content. Therefore, the fluorine atom content is preferably 5.0 to 50 atom%, and more preferably 10 to 25 atom%, based on the total amount of the catalyst in the present disclosure defined as 100 atom%.

The method for fluorinating the metal oxide catalyst is, for example, a method comprising reacting a metal oxide with a fluorinating agent. Specifically, a metal oxide can be fluorinated, for example, by flowing a fluorinating agent through the metal oxide. The metal oxide used in this fluorination can be a metal oxide of the fluorinated metal oxide described above.

The metal oxide catalyst before fluorination preferably has a large pore volume because it can increase the pore volume of the metal oxide catalyst after fluorination and increase the conversion rate of the reaction, and also easily reduce catalyst degradation; and the catalyst degradation can be easily inhibited, even when the isomerization reaction is performed over a long period of time. Specifically, preferable pore volumes of metal oxide catalysts before fluorination are, for example, such that chromium oxide has a pore volume of 0.05 mL/g or more (particularly 0.075 to 1.5 mL/g), alumina has a pore volume of 0.45 mL/g or more (particularly 0.50 to 2.5 mL/g), and silica-alumina has a pore volume of 0.40 mL/g or more (particularly 0.50 to 2.0 mL/g). When the pore volume is within this range, active sites of the catalyst are less likely to be covered with carbon, which is a by-product of the reaction, and the adhered carbon is less likely to inhibit gas diffusion in the pores. Accordingly, the activity of the catalyst can be maintained; that is, it is easy to inhibit catalyst degradation, and the method for producing the catalyst is also simple.

Examples of the fluorinating agent used for this fluorination include hydrofluorocarbons (R23: trifluoromethane, R32: difluoromethane, and R41: monofluoromethane), hydrochlorofluorocarbons (R22: chlorodifluoromethane and R21: dichloromonofluoromethane), chlorofluorocarbons (R13: chlorotrifluoromethane and R11: trichloromonofluoromethane), and the like. Compared with hydrogen fluoride, these fluorinating agents tend to increase the pore volume of the fluorinated metal oxide catalyst, and easily reduce catalyst degradation, and can easily inhibit catalyst degradation even when the isomerization reaction is performed for a long time. These fluorinating agents can be used alone, or in a combination of two or more.

The fluorination conditions are not particularly limited. In view of easily reducing catalyst degradation and easily inhibiting catalyst degradation even when the isomerization reaction is performed over a long time of time, the temperature is preferably 50 to 600°C (particularly 100 to 500°C), the pressure is preferably 0 to 1000 kPa (particularly 0.1 to 500 kPa), and the time is preferably 0.1 to 24 hours (particularly 1 to 12 hours). Under these fluorination conditions, the fluorine atom content is preferably adjusted to fall within the above-described range.

The perfluoroalkyne compound to be produced is, for example, a perfluoroalkyne represented by formula (1):

CR¹₂R²-C≡C-CR³R⁴₂ (1)

(wherein R¹ to R⁴ are the same or different and represent a fluorine atom or a perfluoroalkyl group).

The perfluoroalkyl group represented by R¹ to R⁴ in formula (1) is not particularly limited, and examples include perfluoroalkyl groups having 1 to 6 (particularly 1 to 4) carbon atoms. Specific examples include a trifluoromethyl group, a pentafluoroethyl group, and the like.

In view of, for example, the reaction conversion rate, and the yield and selectivity of the obtained perfluoroalkyne compound, R¹ to R⁴ in formula (1) are all preferably a fluorine atom. R¹ to R⁴ may be the same or different.

Thus, the perfluoroalkyne compound of formula (1) to be produced includes, for example, CF₃C≡CCF₃, CF₃C≡CCF₂CF₃, CF₃C≡CCF(CF₃)₂, CF₃C≡CC(CF₃)₃, CF₃CF₂C≡CCF₂CF₃, CF₃CF₂C≡CCF(CF₃)₂, CF₃CF₂C≡CC(CF₃)₃, (CF₃)₂CFC≡CCF(CF₃)₂, (CF₃)₂CFC≡CC(CF₃)₃, (CF₃)₃CC≡CC(CF₃)₃, and the like.

The perfluoroalkadiene compound as a substrate is preferably, for example, a perfluoroalkadiene represented by formula (2):

CR¹₂=CR²-CR³=CR⁴₂ (2)

(wherein R¹to R⁴ are the same or different and represent a fluorine atom or a perfluoroalkyl group).

The perfluoroalkyl group represented by R¹ to R⁴ in formula (2) is not particularly limited, and examples include perfluoroalkyl groups having 1 to 6 (particularly 1 to 4) carbon atoms. Specific examples include a trifluoromethyl group, a pentafluoroethyl group, and the like.

In view of, for example, the conversion rate of the reaction, and the yield and selectivity of the obtained perfluoroalkyne compound, R¹to R⁴ in formula (2) are all preferably a fluorine atom. R¹to R⁴ may be the same or different.

Examples of the perfluoroalkadiene of formula (2) that satisfies the above conditions include CF₂=CFCF=CF₂, CF₂=CFCF=CFCF₃, CF₃CF=CFCF=CFCF₃, CF(CF₃)=CFCF=CF₂, C (CF₃)₂=CFCF=CF₂, CF (CF₃)=CFCF=CF(CF₃), C(CF₃)₂=CFCF=CF(CF₃), C(CF₃)₂=CFCF=C(CF₃)₂, CF₂=C(CF₃)C(CF₃)=CF₂, and the like. Such perfluoroalkadienes represented by formula (2) can be used alone, or in a combination of two or more.

The perfluoroalkadiene compound as described above can be known or commercially available products. It is also possible to synthesize the perfluoroalkadiene compound in accordance with a conventional method, such as the method disclosed in JP2001-192345A.

### [1-2] Catalyst and Catalyst Production Method (No. 2)

In the present disclosure, catalyst 2 for use in reacting (isomerizing) the perfluoroalkadiene compound to obtain a perfluoroalkyne compound (which may be hereinafter referred to as "isomerization reaction catalyst 2") contains fluorinated metal oxide having a pore volume of 0.35 mL/g or more. This catalyst satisfies the above requirement (D). Conventionally used isomerization reaction catalysts are not optimized in pore volume. It is unclear what catalyst pore volume is used. In the present disclosure, fluorinated metal oxides having specific pore volumes are used, and the pore volume of the fluorinated metal oxide is increased to 0.35 mL/g or more to thereby increase the conversion rate of the reaction and also easily reduce catalyst degradation; accordingly, catalyst degradation can be inhibited, even when the isomerization reaction is performed over a long period of time. Therefore, the use of the catalyst of the present disclosure can reduce the frequency of catalyst replacement, and is thus economical.

The catalyst of the present disclosure is not particularly limited. In view of increasing the conversion rate of the reaction, and easily reducing catalyst degradation and easily inhibiting catalyst degradation even when the isomerization is performed over a long period of time, the catalyst is preferably at least one element that belongs to Groups 3 to 14 in the periodic table of elements, more preferably at least one element that belongs to Groups 4 to 6 and Groups 13 to 14 of the periodic table, and even more specifically at least one element selected from the group consisting of chromium, titanium, silicon, aluminum, zirconium, and the like. The catalyst may contain only one, or two or more, of the above metal elements.

In view of having high activity in the isomerization reaction from the perfluoroalkadiene compound to the perfluoroalkyne compound; and increasing the conversion rate of the reaction as well as easily reducing catalyst degradation and easily inhibiting catalyst degradation, even when the isomerization is performed over a long period of time, the catalyst according to the present disclosure is particularly preferably a fluorinated titanium oxide catalyst, a fluorinated alumina catalyst, a fluorinated silica-alumina catalyst, a fluorinated zirconia catalyst, or the like.

The titanium oxide catalyst to be used may be any catalyst containing titanium dioxide as a main component; and may further contain one or two or more other non-volatile substances, such as metal oxides, hydroxides, sulfates, nitrates, phosphates, and sulfides. The titanium oxide catalyst preferably contains titanium dioxide in an amount of 70% by mass or more.

The titanium dioxide is particularly preferably anatase-type titanium dioxide, and preferably has a specific surface area of 5 to 100 m²/g and a pore volume of 0.2 to 0.4 ml/g. The catalyst is preferably formed into a spherical shape. Specifically, catalysts commercially available under the trade names of, for example, CS-200, CS-300, and CS-950 (produced by Sakai Chemical Industry Co., Ltd.) can be preferably used.

Examples of the alumina catalyst and the silica-alumina catalyst can be those described above in section [1-1] Catalyst and Catalyst Production Method (No. 1).

The zirconia catalyst is also not particularly limited, as long as the catalyst contains zirconium oxide as a main component. The catalyst may contain non-volatile substances, such as other metal oxides, hydroxides, sulfates, nitrates, phosphates, and sulfides. The zirconia catalyst preferably contains zirconia in an amount of 70 mass% or more.

In the present disclosure, a metal oxide catalyst such as those described above is fluorinated to obtain a fluorinated metal oxide catalyst. The fluorinated metal oxide catalyst has strong activity, and the fluorination also easily reduces catalyst degradation; accordingly, even when the isomerization reaction is performed over a long period of time, catalyst degradation can be inhibited by adjustment of the pore volume. Therefore, the metal oxide catalyst is used in the form of a fluorinated metal oxide catalyst. The method for fluorinating the metal oxide catalyst is described later.

The catalyst according to the present disclosure preferably has an increased pore volume, because it is easy to reduce catalyst degradation; and the catalyst degradation can be easily inhibited, even when the isomerization reaction is performed over a long period of time. Specifically, the metal oxide catalyst after fluorination has a pore volume of 0.35 mL/g or more, and preferably 0.40 to 2.0 mL/g. Preferable pore volumes of metal oxide catalysts are, for example, such that fluorinated alumina has a pore volume of 0.35 mL/g or more (particularly 0.40 to 2.0 mL/g), and fluorinated silica alumina has a pore volume of 0.50 mL/g (particularly 0.55 to 2.0 mL/g). When the pore volume is within this range, active sites of the catalyst are less likely to be covered with carbon, which is a by-product of the reaction; and the adhered carbon is less likely to inhibit gas diffusion in the pores. Accordingly, the activity of the catalyst can be maintained; that is, it is easy to inhibit catalyst degradation, and the method for producing the catalyst is also simple.

The degree of fluorination (that is, the fluorine atom content) of the catalyst of the present disclosure, the method of fluorination, the fluorinating agent, the fluorination conditions, and the like can be the same as those described above in section [1-1] Catalyst and Catalyst Production Method (No. 1).

The metal oxide catalyst before fluorination preferably has a large pore volume, because it can increase the pore volume of the metal oxide catalyst after fluorination and increase the conversion rate of the reaction; and also easily reduce catalyst degradation and easily inhibit the catalyst degradation, even when the isomerization reaction is performed over a long period of time. Specifically, the metal oxide catalyst before fluorination preferably has a pore volume of 0.45 mL/g or more, and more preferably 0.50 to 2.5 mL/g. Preferable pore volumes of metal oxide catalysts are, for example, such that alumina has a pore volume of 0.45 mL/g or more (particularly 0.50 to 2.5 mL/g) and silica-alumina has a pore volume of 0.40 mL/g or more (particularly 0.50 to 2.0 mL/g). When the pore volume is within this range, active sites of the catalyst are less likely to be covered with carbon, which is a by-product of the reaction; and the adhered carbon is less likely to inhibit gas diffusion in the pores. Accordingly, the activity of the catalyst can be maintained; that is, it is easy to inhibit catalyst degradation, and the method for producing the catalyst is also simple.

Examples of the perfluoroalkyne compound to be produced and the perfluoroalkadiene compound used as a substrate can be the same as those described above in section [1-1] Catalyst and Catalyst Production Method (No. 1).

### 2. Catalyst Production Method

The method for producing a catalyst of the present disclosure is a method for producing a catalyst for use in reacting a perfluoroalkadiene compound to obtain a perfluoroalkyne compound, and the method comprises the following step (E) or (F):
(E) reacting a metal oxide with at least one compound selected from the group consisting of hydrofluorocarbon, hydrochlorofluorocarbon, and chlorofluorocarbon to fluorinate the metal oxide;
(F) fluorinating at least one metal oxide selected from the group consisting of chromium oxide having a pore volume of 0.10 mL/g or more, alumina having a pore volume of 0.45 mL/g or more, and silica alumina having a pore volume of 0.50 mL/g or more.

### [2-1] Catalyst Production Method (No. 3)

In the present disclosure, the catalyst production method 3 for producing a catalyst for use in reacting (isomerizing) a perfluoroalkadiene compound to obtain a perfluoroalkyne compound (which may be hereinafter referred to as "the isomerization reaction catalyst production method 3") comprises reacting metal oxide with at least one compound selected from the group consisting of hydrofluorocarbon and hydrochlorofluorocarbon to fluorinate the metal oxide. This catalyst satisfies the above requirement (E). Conventionally used isomerization reaction catalysts are not optimized in pore volume. It is unclear what catalyst pore volume is used. In the present disclosure, the metal oxide is fluorinated by a specific compound described above to thereby adjust the pore volume to fall within a predetermined range, increase the conversion rate of the reaction, and also easily reduce catalyst degradation; accordingly, catalyst degradation can be inhibited, even when the isomerization reaction is performed over a long period of time. Therefore, the use of the catalyst of the present disclosure can reduce replacement frequency of the catalyst, and is thus economical.

The metal oxide is not particularly limited. In view of increasing the conversion rate of the reaction, and also easily reducing catalyst degradation and easily inhibiting catalyst degradation even when the isomerization is performed over a long period of time, the metal of the metal oxide is preferably, for example, at least one element that belongs to Groups 3 to 14 of the periodic table; more preferably at least one element that belongs to Groups 4 to 6 and Groups 13 to 14 of the periodic table; and more preferably at least one member selected from the group consisting of chromium, titanium, silicon, aluminum, zirconium, and the like. The metal oxide may contain only one, or two or more, of the above metal elements.

The metal oxide is particularly preferably chromium oxide catalysts, titanium oxide catalysts, alumina catalysts, silica-alumina catalysts, zirconia catalysts, or the like because the fluorination of the metal oxide as described above increases the activity for the isomerization reaction from the perfluoroalkadiene compound to the perfluoroalkyne compound, and increases the conversion rate of the reaction; and also easily reduces catalyst degradation and easily inhibits catalyst degradation, even when the above isomerization reaction is performed over a long period of time.

Examples of chromium oxide catalysts, alumina catalysts, and silica-alumina catalysts can be the same as those described above in section [1-1] Catalyst and Catalyst Production Method (No. 1). Examples of titanium oxide catalysts and zirconia catalysts can be the same as those described above in section [1-2] Catalyst and Catalyst Production Method (No. 2).

The metal oxide catalyst before fluorination as described above preferably has a large pore volume, because it can increase the pore volume of the metal oxide catalyst after fluorination, and increases the conversion rate of the reaction; and also easily reduces catalyst degradation and easily inhibits catalyst reduction, even when the isomerization reaction is performed over a long period of time. Specifically, the metal oxide catalyst before fluorination preferably has a pore volume of 0.45 mL/g or more, more preferably 0.50 to 2.5 mL/g. Preferable pore volumes of metal oxide catalysts before fluorination are, for example, such that chromium oxide has a pore volume of 0.05 mL/g or more (particularly 0.075 to 1.5 mL/g), alumina has a pore volume of 0.45 mL/g or more (particularly 0.50 to 2.5 mL/g), and silica-alumina has a pore volume of 0.40 mL/g or more (particularly 0.50 to 2.0 mL/g). When the pore volume is within this range, active sites of the catalyst are less likely to be covered with carbon, which is a by-product of the reaction; and the adhered carbon is less likely to inhibit gas diffusion in the pores. Accordingly, the activity of the catalyst can be maintained; that is, it is easy to inhibit catalyst degradation, and the method for producing the catalyst is also simple.

In the present disclosure, a metal oxide catalyst such as those described above is fluorinated with a specific compound to obtain a fluorinated metal oxide catalyst. The fluorinated metal oxide catalyst has strong activity and the fluorination also easily reduces catalyst degradation; accordingly, catalyst degradation can be inhibited by adjustment of the pore volume, even when the isomerization reaction is performed over a long period of time. Therefore, the metal oxide catalyst is used in the form of a fluorinated metal oxide catalyst.

In the present disclosure, the method for fluorinating the metal oxide catalyst comprises reacting metal oxide with a fluorinating agent. Specifically, the metal oxide can be fluorinated by, for example, flowing a fluorinating agent through a metal oxide.

In the present disclosure, at least one compound selected from the group consisting of hydrofluorocarbons (R23: trifluoromethane, R32: difluoromethane, R41: monofluoromethane), hydrochlorofluorocarbons (R22: chlorodifluoromethane, R21: dichloromonofluoromethane), and chlorofluorocarbons (R13: chlorotrifluoromethane, R11: trichloromonofluoromethane) is used as the fluorinating agent in this fluorination. As compared with hydrogen fluoride, these fluorinating agents can increase the pore volume of the fluorinated metal oxide catalyst; and can reduce catalyst degradation and inhibit catalyst degradation, even when the isomerization reaction is performed over a long period of time. These fluorinating agents can be used alone, or in a combination of two or more.

The fluorination conditions are not particularly limited. In view of reducing catalyst degradation and easily inhibiting catalyst degradation, even when the isomerization reaction is performed over a long period of time, the temperature is preferably 50 to 600°C (particularly 100 to 500°C), the pressure is preferably 0 to 1000 kPa (particularly 0.1 to 500 kPa), and the isomerization reaction time is preferably 0.1 to 24 hours (particularly 1 to 12 hours).

The catalyst thus obtained has a large pore volume. Therefore, it reduces catalyst degradation and can inhibit catalyst degradation, even when the isomerization reaction is performed over a long period of time. Specifically, the metal oxide catalyst after fluorination preferably has a pore volume of 0.35 mL/g or more, more preferably 0.40 to 2.0 mL/g. Preferable pore volumes of metal oxide catalysts are, for example, such that fluorinated chromium oxide has a pore volume of 0.05 mL/g or more (particularly 0.075 to 1.5 mL/g), fluorinated alumina has a pore volume of 0.35 mL/g or more (particularly 0.40 to 2.0 mL/g), and fluorinated silica alumina has a pore volume of 0.50 ml/g or more (particularly 0.55 to 2.0 mL/g). When the pore volume is within this range, active sites of the catalyst are less likely to be covered with carbon, which is a by-product of the reaction; and the adhered carbon is less likely to inhibit gas diffusion in the pores. Accordingly, the activity of the catalyst can be maintained; that is, it is easy to inhibit catalyst degradation, and the method for producing the catalyst is also simple.

The catalyst thus obtained has a large pore volume. The pore volume can be adjusted by the degree of fluorination of the catalyst; that is, the fluorine atom content. Therefore, the fluorine atom content in the obtained catalyst after fluorination is preferably 5.0 to 50 atom%, more preferably 10 to 25 atom%, per 100 atom% of the total amount of the catalyst after fluorination.

Examples of the perfluoroalkyne compound to be produced and the perfluoroalkadiene compound used as a substrate can be the same as those described above in section [1-1] Catalyst and Catalyst Production Method (No. 1).

### [2-2] Catalyst Production Method (No. 4)

In the present disclosure, the catalyst production method 4 for producing a catalyst for use in reacting (isomerizing) a perfluoroalkadiene compound to obtain a perfluoroalkyne compound (which may be hereinafter referred to as "the isomerization reaction catalyst production method 4") comprises fluorinating at least one metal oxide selected from the group consisting of chromium oxide having a pore volume of 0.10 mL/g or more before fluorination of the metal oxide, alumina having a pore volume of 0.45 mL/g or more before fluorination of the metal oxide, and silica alumina having a pore volume of 0.5 mL/g or more before fluorination of the metal oxide. This catalyst satisfies the above requirement (F). Conventionally used isomerization reaction catalysts are not optimized in pore volume. It is unclear what catalyst pore volume is used. In the present disclosure, metal oxides having a specific pore volume are fluorinated to increase the conversion rate of the reaction and also easily reduce catalyst degradation; accordingly, catalyst degradation can be inhibited, even when the isomerization reaction is performed over a long period of time. Therefore, the use of the catalyst of the present disclosure can reduce replacement frequency of the catalyst, and is thus economical.

Examples of the chromium oxide catalyst, the alumina catalyst, and the silica-alumina catalyst can be the same as those described above in section [1-1] Catalyst and Catalyst Production Method (No. 1).

The metal oxide catalyst before fluorination as described above preferably has a large pore volume because it can increase the pore volume of the metal oxide catalyst after fluorination and increase the conversion rate of the reaction; and can also reduce catalyst degradation and inhibit catalyst degradation, even when the isomerization reaction is performed over a long period of time. Specifically, preferable pore volumes of metal oxide catalysts before fluorination are such that chromium oxide has a pore volume of 0.05 mL/g or more (particularly 0.075 to 1.5 mL/g), alumina has a pore volume of 0.45 mL/g or more (particularly 0.50 to 2.5 mL/g), and silica-alumina has a pore volume of 0.40 mL/g or more (particularly 0.50 to 2.0 mL/g). When the pore volume is smaller than this range, the pores where active sites of the catalyst are present are covered with carbon, which is a by-product of the reaction, or the adhered carbon impairs gas diffusion in the pores, thus reducing the activity of the catalyst, i.e., being likely to cause catalyst degradation. On the other hand, an excessively large pore volume makes the catalyst production method complicated and increases production costs.

In the present disclosure, a metal oxide catalyst such as those described above is fluorinated to obtain a fluorinated metal oxide catalyst. The fluorinated metal oxide catalyst has strong activity, and the fluorination also easily reduces catalyst degradation; accordingly, catalyst degradation can be inhibited by adjustment of the pore volume, even when the isomerization reaction is performed over a long period of time. Therefore, the metal oxide catalyst is used in the form of a fluorinated metal oxide catalyst.

The method of fluorination, the fluorinating agent, the fluorination conditions, and the like can be the same as those described above in section [1-1] Catalyst and Catalyst Production Method (No. 1).

The catalyst thus obtained has a large pore volume. Therefore, it reduces catalyst degradation and the catalyst degradation can be inhibited, even when the isomerization reaction is performed over a long period of time. Specifically, preferable pore volumes of metal oxide catalysts are, for example, such that fluorinated chromium oxide has a pore volume of 0.05 mL/g or more (particularly 0.075 to 1.5 mL/g), fluorinated alumina has a pore volume of 0.35 mL/g (particularly 0.40 to 2.0 mL/g), and fluorinated silica-alumina has a pore volume of 0.50 ml/g or more (particularly 0.55 to 2.0 mL/g). When the pore volume is within this range, active sites of the catalyst are less likely to be covered with carbon, which is a by-product of the reaction; and the adhered carbon is less likely to inhibit gas diffusion in the pores. Accordingly, the activity of the catalyst can be maintained; that is, it is easy to inhibit catalyst degradation, and the catalyst production method is also simple.

The catalyst thus obtained has a large pore volume. The pore volume can be adjusted by the degree of fluorination of the catalyst; that is, the fluorine atom content. Therefore, the fluorine atom content in the obtained catalyst after fluorination is preferably 5.0 to 50 atom%, more preferably 10 to 25 atom%, per 100 atom% of the total amount of the catalyst after fluorination.

Examples of the perfluoroalkyne compound to be produced and the perfluoroalkadiene compound used as a substrate can be the same as those described above in section [1-1] Catalyst and Catalyst Production Method (No. 1).

### 3. Method for Producing Perfluoroalkyne Compound

The method for producing a perfluoroalkyne compound according to the present disclosure comprises reacting a perfluoroalkadiene compound in the presence of a catalyst to obtain a perfluoroalkyne compound, and satisfying one of the following (A) to (G):
(A) the catalyst comprises at least one catalyst selected from the group consisting of catalysts containing a transition metal element, and catalysts containing at least two elements that belong to Groups 3 to 14 of the periodic table of elements;
(B) the catalyst comprises a catalyst containing at least one element that belongs to Groups 3 to 14 of the periodic table, and the contact time between the catalyst and the perfluoroalkadiene compound represented by formula (2) is 30 seconds or less;
(C) the catalyst comprises at least one catalyst selected from the group consisting of fluorinated chromium oxide having a pore volume of 0.08 mL/g or more, fluorinated alumina having a pore volume of 0.35 mL/g or more, and fluorinated silica-alumina having a pore volume of 0.50 mL/g or more;
(D) the catalyst comprises a fluorinated metal oxide having a pore volume of 0.35 mL/g or more;
(E) the catalyst comprises a metal oxide fluorinated by reacting at least one compound selected from the group consisting of hydrofluorocarbon, hydrochlorofluorocarbon, and chlorofluorocarbon with a metal oxide;
(F) the catalyst comprises one or more catalysts obtained by fluorinating at least one metal oxide selected from the group consisting of chromium oxide having a pore volume of 0.10 mL/g or more, alumina having a pore volume of 0.45 mL/g or more, and silica alumina having a pore volume of 0.50 mL/g or more; and
(G) at least a part of the reaction of the perfluoroalkadiene compound from the start to the end of the reaction is performed under conditions in which the reaction system has a water content of 30 ppm by mass or less, based on the mass of the perfluoroalkadiene compound defined as 100 mass%.

Examples of the perfluoroalkyne compound and the perfluoroalkadiene compound can be the same as those described above in section [1-1] Catalyst and Catalyst Production Method (No. 1) .

### [3-1] Method for Producing Perfluoroalkyne Compound (No. 1)

The first method for producing a perfluoroalkyne compound according to the present disclosure comprises reacting a perfluoroalkadiene compound in the presence of a catalyst to obtain a perfluoroalkyne compound, wherein the catalyst comprises at least one catalyst selected from the group consisting of catalysts containing a transition metal element, and catalysts containing at least two elements that belong to Groups 3 to 14 of the periodic table. This production method satisfies the above requirement (A).

Conventionally known methods for producing a perfluoroalkyne compound are limited to methods using catalysts containing only one element that belongs to Group 13 of the periodic table, and not containing any transition metals; or catalysts containing an alkali metal. According to the present disclosure, a catalyst containing a transition metal element, or a catalyst containing at least two elements that belong to Groups 3 to 14 of the periodic table, neither of which has been conventionally used, is used to thereby achieve a high conversion rate of the reaction and obtain a perfluoroalkyne compound with a high yield and a high selectivity, thus broadening the freedom of choice in the perfluoroalkyne compound synthesis. Thus, by using as the catalyst a catalyst containing a transition metal element having high activity, or a catalyst containing at least two elements that belong to Groups 3 to 14 of the periodic table, which has higher activity in the form of a composite oxide, a perfluorocycloalkene compound can be obtained with a high selectivity at a high conversion rate of the reaction. Furthermore, according to the present disclosure, unlike conventional methods, a fluoroalkene compound is less likely to be produced as a by-product, as is described later.

In the present disclosure, a catalyst containing a transition metal element or a catalyst containing at least two elements that belong to Groups 3 to 14 of the periodic table is used as the isomerization reaction catalyst. Such a catalyst is not particularly limited. In view of achieving a particularly high conversion rate of the reaction and obtaining a perfluoroalkyne compound with a high yield and a high selectivity, the catalyst to be used is, for example, preferably a catalyst containing at least one transition metal element that belongs to Groups 4 to 6 of the periodic table, or a catalyst containing at least two elements that belong to Groups 4 to 6 and Groups 13 to 14 of the periodic table. The catalyst is more preferably a catalyst containing chromium, titanium, zirconium, or the like; a catalyst containing silicon and aluminum; or the like.

The isomerization reaction catalyst is preferably an optionally fluorinated chromium oxide catalyst (a chromium oxide catalyst or a fluorinated chromium oxide catalyst), an optionally fluorinated titanium oxide catalyst (a titanium oxide catalyst or a fluorinated titanium oxide catalyst), an optionally fluorinated zirconia catalyst (a zirconia catalyst or a fluorinated zirconia catalyst), or an optionally fluorinated silica-alumina catalyst (a silica-alumina catalyst or a fluorinated silica-alumina catalyst), because these catalysts have high activity in the isomerization reaction from a perfluoroalkadiene compound to a perfluoroalkyne compound; and can also have high activity in the reaction of producing a perfluorocycloalkene compound depending on the reaction conditions, the perfluorocycloalkene compound as well as the perfluoroalkyne compound being expected to be useful as a dry etching gas for semiconductors and also in various applications such as refrigerants, foaming agents, and heat transfer media.

Examples of the chromium oxide catalyst and the silica-alumina catalyst can be the same as those described above in section [1-1] Catalyst and Catalyst Production Method (No. 1). Examples of the titanium oxide catalyst and the zirconia catalyst can be the same as those described above in section [1-2] Catalyst and Catalyst Production Method (No.2).

When the catalyst is fluorinated, the degree of fluorination (fluorine atom content), the method for fluorination, the fluorinating agent, and the fluorination conditions can be those described above in section [1-1] Catalyst and Catalyst Production Method (No. 1).

These isomerization reaction catalyst can be used alone, or in a combination of two or more.

The amount of the isomerization reaction catalyst as described above can be a catalytic amount, and is not particularly limited. In view of achieving a particularly high conversion rate of the reaction and obtaining a perfluoroalkyne compound with a higher yield and a higher selectivity, for example, the weight ratio of the catalyst to the perfluoroalkadiene compound feed rate per hour (W/F) is preferably 0.1 to 200 g·sec/cc, and more preferably 0.5 to 150 g·sec/cc. When two or more isomerization reaction catalysts are used, it is preferable to adjust the total amount of the catalysts to be within the above range. The above W/F specifies the amount of catalyst particularly for the gas phase reaction. Even when the liquid phase reaction is used, the amount of fluoride used can be a catalytic amount, and can be appropriately adjusted.

In the production method of the present disclosure, when the reaction of the perfluoroalkadiene compound is performed, metal nickel (in particular, metal nickel beads), activated carbon, or the like can also be used in addition to the perfluoroalkadiene compound as a substrate and the isomerization reaction catalyst, in an amount to achieve a W/F of 0.1 to 200 g·sec, in particular, 0.5 to 150 g·sec/cc, for the purpose of heat transfer and dilution of the catalyst concentration.

The production method of the present disclosure (in particular, the reaction of a perfluoroalkadiene compound) is preferably performed in a gas phase, in particular in a gas-phase continuous flow process using a fixed bed reactor; however, the method can also be performed in a liquid phase. The reaction in a gas phase can simplify the apparatus, operations, etc., as compared with the reaction in a liquid phase; and can also provide a perfluoroalkyne compound with a higher yield and a higher selectivity than the reaction in the batch system.

In the production method of the present disclosure, the reaction of the perfluoroalkadiene compound is preferably performed with heating. Specifically, heating is preferably performed after the perfluoroalkadiene compound as a substrate and an isomerization reaction catalyst are placed into the reaction system. The heating temperature in this heating is preferably 170°C or more (particularly 170 to 400°C), and more preferably 180 to 280°C, in view of achieving a particularly high conversion rate of the reaction and obtaining a perfluoroalkyne compound with a high yield and a high selectivity.

In the production method of the present disclosure, the contact time (reaction time) between the catalyst and the perfluoroalkadiene compound is not particularly limited. The contact time is preferably 1 to 100 seconds, and more preferably 2 to 30 seconds, in view of achieving a particularly high conversion rate of the reaction and obtaining a perfluoroalkyne compound with a high yield and a high selectivity.

In the production method of the present disclosure, the atmosphere in the reaction of the perfluoroalkadiene compound is not particularly limited. For example, the reaction atmosphere is preferably an inert gas atmosphere (nitrogen gas atmosphere, argon gas atmosphere, etc.).

In the production method of the present disclosure, not only a perfluorocycloalkene compound but also a perfluorocycloalkene compound can be produced. Examples of the perfluorocycloalkene compound include perfluorocycloalkene compounds represented by formula (3): 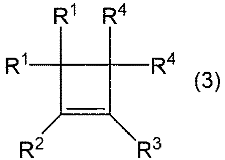 (wherein R¹ to R⁴ are as defined above). The details of the perfluorocycloalkene compound are described later.

Therefore, after completion of the reaction, a purification treatment is performed according to a conventional method, if necessary; and a perfluoroalkyne compound can thus be obtained.

The perfluorocycloalkene compound produced as a by-product by the production method of the present disclosure can be purified according to a conventional method, if necessary; and then used as a substrate to produce a perfluoroalkyne compound. The method and conditions in this process can be the same as those described in Patent Literature (PTL) 2 (JP2014-058488A). Preferable specific examples can also be the same as those described in PTL 2.

Specifically, using the perfluorocycloalkene compound as a substrate, isomerization is performed with an isomerization reaction catalyst to thereby obtain a perfluoroalkyne compound. This step can be performed in a gas phase, particularly in a gas-phase continuous flow process using a fixed bed reactor. Alternatively, this step can also be performed by a batch reaction.

The isomerization reaction catalyst is preferably sodium fluoride that is easy to handle in the atmosphere due to its low hygroscopicity, has high activity, and provides high selectivity. When sodium fluoride itself is used as a catalyst, it may be in powder form. Sodium fluoride in pellet form is preferable for the gas-phase continuous flow reaction. It is also possible to use sodium fluoride supported on a carrier, such as alumina, porous aluminum fluoride, activated carbon, silica, or zeolite. Alternatively, sodium fluoride can be mixed with other components, and then used.

Typically, the isomerization reaction temperature is preferably 200 to 800°C, and more preferably 400 to 600°C.

The perfluorocycloalkene compound as well as the perfluoroalkyne compound obtained as described above can be effectively used as an etching gas for forming state-of-the-art microstructures, such as semiconductors and liquid crystals; and also in various applications, such as building blocks for organic synthesis. The details of the building blocks for organic synthesis are described later.

### [3-2] Method for Producing Perfluoroalkyne Compound (No. 2)

The second method for producing the perfluoroalkyne compound according to the present disclosure is a process comprising reacting a perfluoroalkadiene compound in the presence of a catalyst to obtain a perfluoroalkyne compound, wherein the catalyst comprises at least one catalyst that belongs to Groups 3 to 14 of the periodic table, and the contact time between the catalyst and the perfluoroalkadiene compound is 30 seconds or less. This production method satisfies the above requirement (B).

Conventional methods for producing a perfluoroalkyne compound include methods using a catalyst containing only one element that belongs to Group 13 of the periodic table and not containing any transition metals, such as alumina halide or sodium fluoride; and methods using a catalyst containing an alkali metal. For example, taking, as an example, the case of using a catalyst containing only one element that belongs to Group 13 of the periodic table and not containing any transition metals, there have been only cases in which the reaction time is long; i.e., 32 seconds or longer. According to the present disclosure, by using a catalyst containing at least one element that belongs to Groups 3 to 14 of the periodic table and shortening the reaction time to 30 seconds or less to thereby achieve a high conversion rate of the reaction and obtain a perfluoroalkyne compound with a high yield and a high selectivity, thus broadening the freedom of choice in the perfluoroalkyne compound synthesis. According to this method, due to the short reaction time, the reaction can proceed economically. Furthermore, according to the present disclosure, unlike conventional methods, a fluoroalkene compound is less likely to be produced as a by-product, as is described later.

In the present disclosure, a catalyst containing at least one element that belongs to Groups 3 to 14 of the periodic table is used as an isomerization reaction catalyst. Such a catalyst is not particularly limited. In view of achieving a particularly high conversion rate of the reaction and obtaining a perfluoroalkyne compound with a higher yield and a higher selectivity, catalysts containing a transition metal element and catalysts containing at least two elements that belong to Groups 3 to 14 of the periodic table are preferable. Catalysts containing at least one transition metal element that belongs to Groups 4 to 6 of the periodic table and catalysts containing at least two elements that belong to Groups 4 to 6 and Groups 13 to 14 of the periodic table are more preferable. Catalysts containing chromium, titanium, and zirconium; catalysts containing silicon and aluminum; and the like are even more preferable.

The isomerization reaction catalyst is preferably an optionally fluorinated chromium oxide catalyst (a chromium oxide catalyst or a fluorinated chromium oxide catalyst), an optionally fluorinated titanium oxide catalyst (a titanium oxide catalyst or a fluorinated titanium oxide catalyst), an optionally fluorinated zirconia catalyst (a zirconia catalyst or a fluorinated zirconia catalyst), or an optionally fluorinated silica-alumina catalyst (a silica-alumina catalyst or a fluorinated silica-alumina catalyst), because these catalysts have high activity in the isomerization reaction from a perfluoroalkadiene compound to a perfluoroalkyne compound and can also have high activity in the reaction of producing a perfluorocycloalkene compound depending on the reaction conditions, the perfluorocycloalkene compound as well as the perfluoroalkyne compound being expected to be useful as a dry etching gas for semiconductors and also in various applications such as refrigerants, foaming agents, heat transfer media, and the like. In this embodiment, an optionally fluorinated alumina catalyst can also be used as a catalyst containing one element that belongs to Group 13 of the periodic table.

Examples of the chromium oxide catalyst, the alumina catalyst and the silica-alumina catalyst can be the same as those described above in section [1-1] Catalyst and Catalyst Production Method (No. 1). Examples of the titanium oxide catalyst and the zirconia catalyst can be the same as those described above in section [1-2] Catalyst and Catalyst Production Method (No. 2).

These isomerization reaction catalysts can be used alone, or in a combination of two or more.

When the catalyst is fluorinated, the degree of fluorination (fluorine atom content), the method of fluorination, the fluorinating agent, and the fluorination conditions can be those as described above in section [1-1] Catalyst and Catalyst Production Method (No. 1).

The amount of the isomerization reaction catalyst as described above can be the same as those described above in section [3-1] Method for Producing Perfluoroalkyne Compound (No. 1) .

In the production method of the present disclosure, when the reaction of the perfluoroalkadiene compound is performed, metal nickel (in particular, metal nickel beads), activated carbon, or the like can also be used in addition to the perfluoroalkadiene compound as a substrate and the isomerization reaction catalyst, in an amount to achieve a W/F of 0.1 to 200 g·sec, in particular, 0.5 to 150 g·sec/cc, for the purpose of heat transfer and dilution of the catalyst concentration.

The production method of the present disclosure (in particular, the reaction of a perfluoroalkadiene compound) can be performed in a liquid phase, but is preferably performed in a gas phase, in particular in a gas-phase continuous flow process using a fixed bed reactor. This can simplify the apparatus, operations, etc., as compared with the reaction in a liquid phase; and can also provide a perfluoroalkyne compound with a higher yield and a higher selectivity than the reaction in the batch system.

In the production method of the present disclosure, the reaction temperature and the reaction atmosphere in the reaction of the perfluoroalkadiene compound can be the same as those described above in section [3-1] Method for Producing Perfluoroalkyne Compound (No. 1).

In the production method of the present disclosure, the contact time (reaction time) between the catalyst and the perfluoroalkadiene compound is 30 seconds or less, and preferably 25 seconds or less. When the contact time (reaction time) is more than 30 seconds, the yield of the perfluoroalkyne compound is reduced. The lower limit of the contact time (reaction time) is not particularly limited, but is usually 1 second.

In the production method of the present disclosure, not only a perfluorocycloalkene compound but also a perfluorocycloalkene compound can be produced. Examples of the perfluorocycloalkene compound include perfluorocycloalkene compounds represented by formula (3): 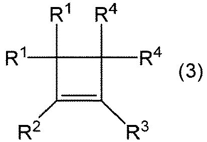 (wherein R¹ to R⁴ are as defined above). The details of the perfluorocycloalkene compound are described later.

Therefore, after completion of the reaction, a purification treatment is performed according to a conventional method, if necessary; and a perfluoroalkyne compound can thus be obtained.

The perfluorocycloalkene compound produced as a by-product in the production method of the present disclosure can be purified according to a conventional method, if necessary, and then used as a substrate to produce a perfluoroalkyne compound. The method and conditions for producing the perfluoroalkyne compound in this process can be the same as those described above in section [3-1] Method for Producing Perfluoroalkyne Compound (No. 1) .

The perfluorocycloalkene compound as well as the perfluoroalkyne compound thus obtained can be effectively used as an etching gas for forming state-of-the-art microstructures, such as semiconductors and liquid crystals; and also in various applications, such as building blocks for organic synthesis. The details of the building block for organic synthesis are described later.

### [3-3] Method for Producing Perfluoroalkyne Compound (No. 3)

The third method for producing a perfluoroalkyne compound according to the present disclosure is a process comprising reacting a perfluoroalkadiene compound in the presence of a catalyst to obtain a perfluoroalkyne compound, and satisfying any one of the following (C) to (F):
(C) the catalyst comprises at least one catalyst selected from the group consisting of fluorinated chromium oxide having a pore volume of 0.08 mL/g or more, fluorinated alumina having a pore volume of 0.35 mL/g or more, and fluorinated silica-alumina having a pore volume of 0.50 mL/g or more;
(D) the catalyst comprises a fluorinated metal oxide having a pore volume of 0.35 mL/g or more;
(E) the catalyst comprises a metal oxide fluorinated by reacting a metal oxide with at least one compound selected from the group consisting of hydrofluorocarbon, hydrochlorofluorocarbon, and chlorofluorocarbon; and
(F) the catalyst comprises one or more catalysts obtained by fluorinating at least one metal oxide selected from the group consisting of chromium oxide having a pore volume of 0.10 mL/g or more, alumina having a pore volume of 0.45 mL/g or more, and silica alumina having a pore volume of 0.50 mL/g or more.

Requirement (C) means that the isomerization reaction catalyst 1 of the present disclosure described above in section [1-1] Catalyst and Catalyst Production Method (No. 1) is used. Requirement (D) means that the isomerization reaction catalyst 2 of the present disclosure described above in section [1-2] Catalyst and Catalyst Production Method (No. 2) is used. Requirement (E) means that the catalyst obtained by the isomerization reaction catalyst production method 3 described above in section [2-1] Catalyst Production Method (No. 3) is used. Requirement (F) means that the catalyst obtained by the isomerization reaction catalyst production method 4 described above in section [2-2] Catalyst and Catalyst Production Method (No. 4) is used. The catalysts of the present disclosure can be used alone, or in a combination of two or more.

In the present disclosure, the catalyst of the present disclosure as described above is used to thereby increase the conversion rate of the reaction, and also easily reduce catalyst degradation; accordingly, even when the isomerization reaction is performed over a long period of time, catalyst degradation can be inhibited. Therefore, the use of the catalyst of the present disclosure can reduce replacement frequency of the catalyst, and is thus economical.

The amount of the catalyst of the present disclosure as described above can be a catalyst amount, and is not particularly limited. In view of achieving a particularly high conversion rate of the reaction, and easily reducing catalyst degradation and easily inhibiting catalyst degradation even when the isomerization reaction is performed over a long period of time, the weight ratio of the catalyst to the perfluoroalkadiene compound feed rate per hour (W/F) is preferably 0.1 to 200 g·sec/cc, and more preferably 0.5 to 150 g·sec/cc. When two or more isomerization reaction catalysts are used, it is preferable to adjust the total amount of the catalysts to be within the above range. The above W/F specifies the amount of catalyst, particularly for the gas phase reaction. Even when the liquid phase reaction is used, the amount of fluoride used can be a catalytic amount, and can be appropriately adjusted.

In the production method of the present disclosure, metal nickel (in particular, metal nickel beads), activated carbon, or the like can also be used in addition to the perfluoroalkadiene compound as a substrate and the isomerization reaction catalyst, in an amount to achieve a W/F of 0.1 to 200 g·sec, in particular, 0.5 to 150 g·sec/cc, for the purpose of heat transfer and dilution of the catalyst concentration.

In the production method of the present disclosure, the isomerization reaction can be performed in a liquid phase, but is preferably performed in a gas phase, in particular in a gas-phase continuous flow process using a fixed bed reactor. This can simplify the apparatus, operations, and the like, as compared with the reaction in a liquid phase; and can also provide a perfluoroalkyne compound with a higher yield and a higher selectivity than the reaction in the batch system. Furthermore, it is easy to reduce catalyst degradation; and even when the isomerization reaction is performed over a long period of time, catalyst degradation can be easily inhibited.

In the production method of the present disclosure, the isomerization reaction is preferably performed with heating. Specifically, heating is preferably performed after the perfluoroalkadiene compound as a substrate and the catalyst of the present disclosure are placed into the reaction system. The heating temperature in this heating is preferably 170°C or more (particularly 170 to 400°C), and more preferably 180 to 280°C, in view of achieving a particularly high conversion rate of the reaction, easily reducing catalyst degradation, and easily inhibiting catalyst degradation, even when the isomerization reaction is performed over a long period of time.

In the production method of the present disclosure, the reaction time in the isomerization reaction is not particularly limited. Since the production method of the present disclosure easily reduces catalyst degradation and inhibits catalyst degradation even when the isomerization reaction is performed over a long period of time, the method is suitable for long-term reactions. The reaction time is preferably 10 to 200 hours, and more preferably 20 to 100 hours.

In the production method of the present disclosure, the atmosphere in the isomerization reaction is not particularly limited. For example, the reaction atmosphere is preferably an inert gas atmosphere (nitrogen gas atmosphere, argon gas atmosphere, etc.).

After completion of the reaction, if necessary, a purification treatment is performed according to a conventional method, and a perfluoroalkyne compound can thus be obtained.

The perfluoroalkyne compound obtained as described above can be effectively used as an etching gas for forming state-of-the-art microstructures, such as semiconductors and liquid crystals; and also in various applications, such as building blocks for organic synthesis. The details of the building block for organic synthesis will be described later.

### [3-4] Method for Producing Perfluoroalkyne Compound (No. 4)

The fourth method for producing a perfluoroalkyne compound according to the present disclosure is a method comprising reacting a perfluoroalkadiene compound in the presence of a catalyst to obtain a perfluoroalkyne compound, wherein at least a part of the reaction of the perfluoroalkadiene compound from the start to the end of the reaction is performed under conditions in which the water content of the reaction system is 30 ppm by mass or less, based on the mass of the perfluoroalkadiene compound (defined as 100 mass%).

As a method for producing a perfluoroalkyne compound, a method comprising reacting and isomerizing a perfluoroalkadiene compound in the presence of a catalyst is known. However, a method that is capable of reducing catalyst degradation is not known. According to the present disclosure, the reaction system has a water content of 30 ppm by mass or less, based on the mass of the perfluoroalkadiene compound (100 mass%), in at least a part of the reaction from the start to the end of the reaction to thereby reduce catalyst degradation and maintain a high conversion rate of the reaction; accordingly, catalyst degradation can also be inhibited, even when the isomerization reaction is performed for a long period of time. Further, since the catalyst degradation rate also varies depending on the weight ratio of the catalyst to the perfluoroalkadiene compound feed rate per hour (W/F), catalyst degradation can be further inhibited by increasing the W/F; i.e., by delaying the perfluoroalkadiene compound feed rate. Therefore, when the production method according to the present disclosure is used, replacement frequency of the catalyst can be reduced, and the production method is thus economical.

As is described in detail later, according to the present disclosure, the water content of the reaction system is controlled to a low level. This can effectively prevent water from covering active sites of the catalyst, and causing catalyst degradation. That is, no matter what type of catalyst is used, the method according to the present disclosure can inhibit water from covering active sites of the catalyst, and can effectively reduce catalyst degradation. The isomerization reaction catalyst is not particularly limited. In view of reducing the water content of the reaction system to thereby easily reduce catalyst degradation and easily inhibit catalyst degradation even when the isomerization reaction is performed over a long period of time, the catalyst is, for example, preferably a catalyst containing at least one element that belongs to Groups 3 to 14 of the periodic table; more preferably a catalyst containing at least one element that belongs to Groups 4 to 6 and Groups 13 to 14 of the periodic table; and even more preferably a catalyst containing at least one element selected from the group consisting of chromium, titanium, silicon, and aluminum. The catalyst may contain only one, or two or more of the metal elements described above.

In view of having high activity in the isomerization reaction from a perfluoroalkadiene compound to a perfluoroalkyne compound, easily reducing catalyst degradation by reducing the water content of the reaction system, and easily inhibiting the degradation even when the isomerization reaction is performed over a long period of time, the isomerization reaction catalyst is particularly preferably an optionally fluorinated chromium oxide catalyst (a chromium oxide catalyst or a fluorinated chromium oxide catalyst), an optionally fluorinated titanium oxide catalyst (a titanium oxide catalyst or a fluorinated titanium oxide catalyst), an optionally fluorinated alumina catalyst (an alumina catalyst or a fluorinated alumina catalyst), or an optionally fluorinated silica-alumina catalyst (a silica-alumina catalyst or a fluorinated silica-alumina catalyst).

Examples of the chromium oxide catalyst, the alumina catalyst, and the silica-alumina catalyst can be the same as those described above in section [1-1] Catalyst and Catalyst Production Method (No. 1). Examples of the titanium oxide catalyst and the zirconia catalyst can be the same as those described above in section [1-2] Catalyst and Catalyst Production Method (No. 2).

Such isomerization reaction catalysts can be used alone, or in a combination of two or more.

When the catalyst is fluorinated, the degree of fluorination (fluorine atom content), the method for fluorination, the fluorinating agent, and the fluorination conditions can be the same as those described above in section [1-1] Catalyst and Catalyst Production Method (No. 1).

The amount of the isomerization reaction catalyst as described above can be the same as those described above in section [3-1] Method for Producing Perfluoroalkyne Compound (No. 1) .

In the production method of the present disclosure, the isomerization reaction (the reaction of a perfluoroalkadiene compound) can also be performed using metal nickel (in particular, metal nickel beads), activated carbon, or the like in addition to the perfluoroalkadiene compound as a substrate and the isomerization reaction catalyst, in an amount to achieve a W/F of 0.1 to 200 g·sec, in particular, 0.5 to 150 g·sec/cc, for the purpose of heat transfer and dilution of the catalyst concentration.

In the production method of the present disclosure, at least a part of the isomerization reaction from the start to the end of the reaction (the reaction of a perfluoroalkadiene compound) is performed under conditions in which the reaction system has a water content of 30 ppm by mass or less, preferably 20 ppm by mass or less, based on the mass of the perfluoroalkadiene compound (defined as 100 mass%). The lower limit of the water content can be 1 ppm by mass. In the present disclosure, the water content of the reaction system can be adjusted, for example, by supplying a perfluoroalkadiene compound having a specific water content while the reaction atmosphere is an inert gas atmosphere, as described later.

In the present disclosure, the water content of the reaction system is 30 ppm by mass or less, as described above. The reaction system may have a water content of 30 ppm by mass or less at the beginning of the reaction, during the reaction, or at the end of the reaction. That is, it is sufficient if the water content of the reaction system is 30 ppm by mass or less in at least a part of the reaction from the start to the end of the reaction (at least at any timing). In view of more efficiently inhibiting catalyst degradation, the reaction system preferably has a water content of 30 ppm by mass or less (in particular, 20 ppm by mass or less) in at least at the start of the reaction. It is particularly preferable that the water content of the reaction system is maintained at 30 ppm by mass or less (in particular, 20 ppm by mass or less) throughout the reaction, from the start to the end of the reaction.

In the production method of the present disclosure, the reaction atmosphere in the isomerization reaction (reaction of the perfluoroalkadiene compound) is not particularly limited, as long as the water content in the reaction system falls within the above range. The reaction atmosphere is, for example, preferably an inert gas atmosphere (nitrogen gas atmosphere, argon gas atmosphere, etc.). When the reaction atmosphere is an inert gas atmosphere, a water content of 30 ppm by mass or less in the reaction system can be paraphrased as the perfluoroalkadiene compound used as a substrate substantially having a water content of 30 ppm by mass or less. That is, when the reaction atmosphere is an inert gas atmosphere, the production method of the present disclosure can be paraphrased as a method comprising reacting a perfluoroalkadiene compound having a water content of 30 ppm by mass or less in the presence of a catalyst to obtain a perfluoroalkyne compound. In this case as well, the perfluoroalkyne compound may have a water content of 30 ppm by mass or less at the beginning of the reaction, more specifically, immediately before being brought into contact with the catalyst; the perfluoroalkyne compound in the reaction system may have a water content of 30 ppm by mass or less at some point during the reaction; or the perfluoroalkyne compound may have a water content of 30 ppm by mass or less immediately after the end of the reaction. That is, it is sufficient if the perfluoroalkyne compound has a water content of 30 ppm by mass or less in at least a part of the reaction from the start to the end of the reaction (at least at any timing). The perfluoroalkyne compound preferably has a water content of 30 ppm by mass or less at the start to reaction. It is particularly preferable that the perfluoroalkyne compound has a water content of 30 ppm by mass or less throughout the reaction, from the start to the end of the reaction.

In the production method of the present disclosure, the isomerization reaction (the reaction of a perfluoroalkadiene compound) can be performed in a liquid phase, but is preferably performed in a gas phase, in particular in a gas-phase continuous flow process using a fixed bed reactor. This can simplify the apparatus, operations, etc., as compared with the reaction in a liquid phase; and can also provide a perfluoroalkyne compound with a higher yield and a higher selectivity than the reaction in the batch system. Furthermore, reducing the water content of the reaction system makes it easy to reduce catalyst degradation, and the catalyst degradation can be easily inhibited, even when the isomerization reaction is performed over a long period of time.

In the production method of the present disclosure, the reaction temperature in the reaction of the perfluoroalkadiene compound can be the same as those described above in section [3-1] Method for Producing Perfluoroalkyne Compound (No. 1).

In the production method of the present disclosure, the reaction time of the isomerization reaction (reaction of the perfluoroalkadiene compound) is not particularly limited. Since the production method of the present disclosure easily reduces catalyst degradation and inhibits catalyst degradation even when the isomerization reaction is performed over a long period of time, the method is suitable for long-term reactions. The reaction time is preferably 10 to 200 hours, and more preferably 20 to 100 hours.

After completion of the reaction, a purification treatment is performed according to a conventional method, if necessary; and a perfluoroalkyne compound can thus be obtained.

The perfluoroalkyne compound obtained as described above can be effectively used as an etching gas for forming state-of-the-art microstructures, such as semiconductors and liquid crystals; and also in various applications, such as building blocks for organic synthesis. The details of the building block for organic synthesis are described later.

### 4. Perfluorocycloalkene Composition

The perfluoroalkyne compounds can be obtained as described above. However, the use of the production method described above in section "[3-1] Method for Producing Perfluoroalkyne Compound (No. 1)" or section "[3-2] Method for producing Perfluoroalkyne Compound (No. 2)" can possibly also produce a product in the form of a perfluoroalkyne composition comprising a perfluoroalkyne compound and a perfluorocycloalkene compound, as described above. The use of the production method described above in section "[3-3] Method for Producing Perfluoroalkyne Compound (No. 3)" or section "[3-4] Method for Producing Perfluoroalkyne Compound (No. 4)" can achieve exceedingly high selectivity of the obtained perfluoroalkyne compound, and can exceedingly reduce the content of other additional compounds in the product.

When the perfluoroalkyne compound is obtained in the form of a perfluoroalkyne composition, the perfluoroalkyne compound is preferably perfluoroalkyne represented by formula (1) :

CR¹₂R²-C≡C-CR³R⁴₂ (1),

wherein R¹ to R⁴ are as defined above, and
the perfluorocycloalkene compound is preferably a perfluorocycloalkene compound represented by formula (3): 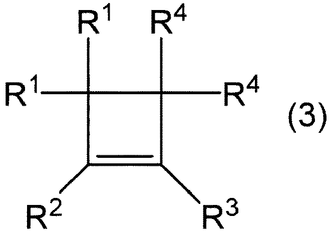 wherein R¹ to R⁴ are as defined above.

In the perfluoroalkyne composition of the present disclosure, the perfluoroalkyne compounds can be used alone, or in a combination of two or more.

Examples of perfluorocycloalkene compounds include the following compounds.

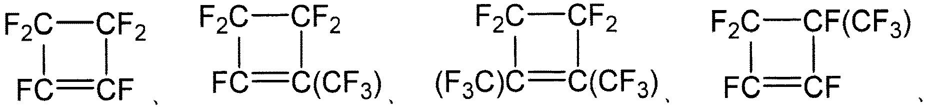

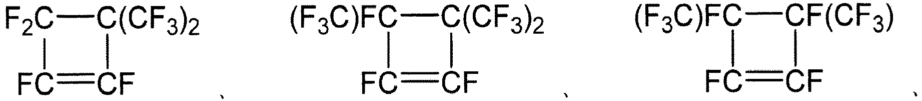

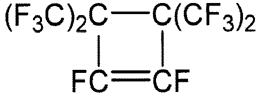

The perfluorocycloalkene compounds can be used alone, or in a combination of two or more.

In the perfluoroalkyne composition of the present disclosure, the content of the perfluoroalkyne compound is preferably 40 to 99.999 mol%, more preferably 50 to 99.998 mol%, and even more preferably 60 to 99.997 mol%, based on the total amount of the perfluoroalkyne composition of the present disclosure defined as 100 mol%. Likewise, the content of the perfluorocycloalkene compound is preferably 0.001 to 60 mol%, more preferably 0.002 to 50 mol%, and even more preferably 0.003 to 40 mol%, based on the total amount of the perfluoroalkyne composition of the present disclosure defined as 100 mol%.

In addition to the perfluoroalkyne compounds and the perfluorocycloalkene compounds above, the production method of the present disclosure can also produce, for example, a perfluoroalkene compound represented by formula (4A):

CR¹₂=CR²-CFR³-CFR⁴₂ ( 4A),

wherein R¹ to R⁴ are as defined above, and
a fluoroalkene compound represented by formula (4B):

CFR¹₂-CR²=CH-CFR⁴₂ (4B),

wherein R¹ to R⁴ are as defined above.
However, according to the production method of the present disclosure, a fluoroalkene compound represented by formula (4B) is unlikely to be obtained as a by-product.

Therefore, the perfluoroalkyne composition of the present disclosure can also comprise a perfluoroalkene compound represented by formula (4A) and a fluoroalkene compound represented by formula (4B). When a perfluoroalkene compound represented by formula (4A) is contained in the perfluoroalkyne composition of the present disclosure, the content is preferably 0.0005 to 0.5 mol%, and more preferably 0.001 to 0.3 mol%, based on the total amount of the perfluoroalkyne composition of the present disclosure defined as 100 mol%. Further, when a fluoroalkene compound represented by formula (4B) is contained in the perfluoroalkyne composition of the present disclosure, the content is preferably 0 to 0.3 mol%, and more preferably 0.01 to 0.28 mol%, based on the total amount of the perfluoroalkyne composition of the present disclosure defined as 100 mol%, since the fluoroalkene compound represented by formula (4B) is unlikely to be obtained as a by-product by the production method of the present disclosure.

According to the production method of the present disclosure, the perfluoroalkyne compound can be obtained, in particular, with a high yield and a high selectivity as described above, even when it is obtained as a perfluoroalkyne composition. Therefore, the components other than the perfluoroalkyne compound in the perfluoroalkyne composition can be reduced, and the labor required for purification to obtain the perfluoroalkyne compound can thus be reduced.

As in the case of the perfluoroalkyne compound described above alone, the perfluoroalkyne composition of the present disclosure can also be effectively used for various applications, such as an etching gas used for forming state-of-the-art microstructures (e.g., semiconductors and liquid crystals), and a building block for organic synthesis. The term "building block for organic synthesis" means a substance that can serve as a precursor for a compound having a highly reactive skeleton. For example, the perfluorocycloalkene composition of the present disclosure can be converted into a substance that can serve as a detergent or a fluorine-containing medicinal intermediate by reacting with a fluorine-containing organosilicon compound, such as CF₃Si(CH₃)₃, and introducing fluoroalkyl, such as a CF₃ group.

The embodiments according to the present disclosure are described above; however, various changes in forms and details can be made without departing from the spirit and scope of the claims.

### Examples

The features of the present disclosure are clarified below with reference to Examples. However, the present disclosure is not limited to the Examples.

### Example 1: Requirement (A) or (B)

The following catalysts were used in Example 1 below.
Chromia catalyst: Cr₂O₃
Fluorinated chromia catalyst (1): Cr₂O₃ was fluorinated by allowing hydrogen fluoride to flow through under atmospheric pressure at 100 to 460°C for 3 to 4 hours.
Titania catalyst: TiO₂
Fluorinated titania catalyst: TiO₂ was fluorinated by allowing hydrogen fluoride to flow through under atmospheric pressure at room temperature to 300°C for 3 to 4 hours.
Fluorinated zirconia catalyst: ZrO₂ was fluorinated by allowing hydrogen fluoride to flow through under atmospheric pressure at room temperature to 400°C for 3 to 4 hours.
Silica-alumina catalyst: SiO₂/Al₂O₃ = 80/10 to 60/20 (mass ratio).

### Example 1-1: Fluorinated Chromia Catalyst; W/F = 30.0 g·sec/cc; 200°C; 23.1 sec

As a catalyst, the fluorinated chromia catalyst (1) (chromia fluorinated with hydrogen fluoride) was filled in a metal tubular reactor. The reaction tube was heated to 200°C, and hexafluorobutadiene (CF₂CF=CFCF₂) was fed to the reaction tube to achieve a W/F of 30.0 g·sec/cc to allow a reaction to proceed in a gas-phase continuous flow process for 23.1 seconds. One hour after the completion of the reaction, the gas flowing out of the reaction tube was analyzed by gas chromatography, which revealed that the conversion rate was 100 mol%, and the selectivity of each component was as follows: 99.7 mol% for 1,1,1,4,4,4-hexafluoro-2-butyne (CF₃C≡CCF₃), 0.162 mol% for 1,2,3,3,4,4-hexafluoro-1-cyclobutene (c-C₄F₆), 0.0356 mol% for 1,1, 2, 3, 3, 4, 4, 4-octafluoro-1-butene (CF₂=CFCF₂CF₃) (the total amount of the E-isomer and Z-isomer), 0.0947 mol% for 1,1,1,2,4,4,4-heptafluoro-2-butene (CF₃CF=CHCF₃) (the total amount of the E-isomer and Z-isomer), and 0.0182 mol% for the total of other by-products.

### Example 1-2: Fluorinated Chromia Catalyst; W/F = 30.0 g·sec/cc; 250°C; 23.1 sec

The reaction was carried out in the same manner as in Example 1-1, except that the reaction was carried out at a heating temperature of 250°C for 23.1 seconds. One hour after the completion of the reaction, the gas flowing out of the reaction tube was analyzed by gas chromatography, which revealed that the conversion rate was 100 mol%, and the selectivity of each component was as follows: 97.2 mol% for 1,1,1,4,4,4-hexafluoro-2-butyne (CF₃C≡CCF₃), 2.47 mol% for 1, 2, 3, 3, 4, 4-hexafluoro-1-cyclobutene (c-C₄F₆), 0.0871 mol% for 1,1,2,3,3,4,4,4-octafluoro-1-butene (CF₂=CFCF₂CF₃) (the total amount of the E-isomer and Z-isomer), 0.262 mol% for 1,1,1,2,4,4,4-heptafluoro-2-butene (CF₃CF=CHCF₃) (the total amount of the E-isomer and Z-isomer), and 0.0194 mol% for the total of other by-products.

### Example 1-3: Fluorinated Chromia Catalyst; W/F = 90.0 g·sec/cc; 200°C; 69.3 sec

The reaction was carried out in the same manner as in Example 1-1, except that the reaction was carried out with a W/F of 90.0 g·sec/cc for 69.3 seconds. One hour after the completion of the reaction, the gas flowing out of the reaction tube was analyzed by gas chromatography, which revealed that the conversion rate was 100 mol%, and the selectivity of each component was as follows: 99.7 mol% for 1,1,1,4,4,4-hexafluoro-2-butyne (CF₃C≡CCF₃), 0.118 mol% for 1,2,3,3,4,4-hexafluoro-1-cyclobutene (c-C₄F₆), 0.0254 mol% for 1,1,2,3,3,4,4,4-octafluoro-1-butene (CF₂=CFCF₂CF₃) (the total amount of the E-isomer and Z-isomer), 0.0911 mol% for 1,1,1,2,4,4,4-heptafluoro-2-butene (CF₃CF=CHCF₃) (the total amount of the E-isomer and Z-isomer), and 0.0162 mol% for the total of other by-products.

### Example 1-4: Fluorinated Titania Catalyst; W/F = 30.0 g·sec/cc; 200°C; 32.5 sec

The reaction was carried out in the same manner as in Example 1-1, except that the reaction was carried out for 32 seconds by using the fluorinated titania catalyst (titania fluorinated with hydrogen fluoride) as the catalyst. One hour after the completion of the reaction, the gas flowing out of the reaction tube was analyzed by gas chromatography, which revealed that the conversion rate was 99.0 mol%, and the selectivity of each component was as follows: 99.3 mol% for 1,1,1,4,4,4-hexafluoro-2-butyne (CF₃C≡CCF₃), 0.354 mol% for 1,2,3,3,4,4-hexafluoro-1-cyclobutene (c-C₄F₆), 0.0595 mol% for 1,1, 2, 3, 3, 4, 4, 4-octafluoro-1-butene (CF₂=CFCF₂CF₃) (the total amount of the E-isomer and Z-isomer), 0.0341 mol% for 1,1,1,2,4,4,4-heptafluoro-2-butene (CF₃CF=CHCF₃) (the total amount of the E-isomer and Z-isomer), and 0.213 mol% for the total of other by-products.

### Example 1-5: Fluorinated Titania Catalyst; W/F = 30.0 g·sec/cc; 250°C; 32.5 sec

The reaction was carried out in the same manner as in Example 1-1, except that the reaction was carried out at a heating temperature of 250°C for 32.5 seconds by using the fluorinated titania catalyst (titania fluorinated with hydrogen fluoride) as the catalyst. One hour after the completion of the reaction, the gas flowing out of the reaction tube was analyzed by gas chromatography, which revealed that the conversion rate was 99.9 mol%, and the selectivity of each component was as follows: 23.7 mol% for 1,1,1,4,4,4-hexafluoro-2-butyne (CF₃C≡CCF₃), 76.0 mol% for 1, 2, 3, 3, 4, 4-hexafluoro-1-cyclobutene (c-C₄F₆), 0.00110 mol% for 1, 1, 2, 3, 3, 4, 4, 4-octafluoro-1-butene (CF₂=CFCF₂CF₃) (the total amount of the E-isomer and Z-isomer), 0.00421 mol% for 1,1,1,2,4,4,4-heptafluoro-2-butene (CF₃CF=CHCF₃) (the total amount of the E-isomer and Z-isomer), and 0.295 mol% for the total of other by-products.

### Example 1-6: Fluorinated Titania Catalyst; W/F = 30.0 g·sec/cc; 300°C; 32.5 sec

The reaction was carried out in the same manner as in Example 1-1, except that the reaction was carried out at a heating temperature of 300°C for 32.5 seconds by using the fluorinated titania catalyst (titania fluorinated with hydrogen fluoride) as the catalyst. One hour after the completion of the reaction, the gas flowing out of the reaction tube was analyzed by gas chromatography, which revealed that the conversion rate was 100 mol%, and the selectivity of each component was as follows: 44.5 mol% for 1,1,1,4,4,4-hexafluoro-2-butyne (CF₃C≡CCF₃), 55.1 mol% for 1, 2, 3, 3, 4, 4-hexafluoro-1-cyclobutene (c-C₄F₆), 0.00601 mol% for 1, 1, 2, 3, 3, 4, 4, 4-octafluoro-1-butene (CF₂=CFCF₂CF₃) (the total amount of the E-isomer and Z-isomer), 0.00200 mol% for 1,1,1,2,4,4,4-heptafluoro-2-butene (CF₃CF=CHCF₃) (the total amount of the E-isomer and Z-isomer), and 0.392 mol% for the total of other by-products.

### Example 1-7: Fluorinated Zirconia Catalyst; W/F= 30.0 g·sec/cc; 250°C; 14.9 sec

The reaction was carried out in the same manner as in Example 1-1, except that the reaction was carried out at a heating temperature of 250°C for 14.9 seconds by using the fluorinated zirconia catalyst (zirconia fluorinated with hydrogen fluoride) as the catalyst. One hour after the completion of the reaction, the gas flowing out of the reaction tube was analyzed by gas chromatography, which revealed that the conversion rate was 70.1 mol%, and the selectivity of each component was as follows: 4.18 mol% for 1, 2, 3, 3, 4, 4-hexafluoro-1-cyclobutene (c-C₄F₆), 94.1 mol% for 1,1,1,4,4,4-hexafluoro-2-butyne (CF₃C≡CCF₃), 0.00100 mol% for 1, 1, 2, 3, 3, 4, 4, 4-octafluoro-1-butene (CF₂=CFCF₂CF₃) (the total amount of the E-isomer and Z-isomer), 0.198 mol% for 1,1,1,2,4,4,4-heptafluoro-2-butene (CF₃CF=CHCF₃) (the total amount of the E-isomer and Z-isomer), and 0.0760 mol% for the total of other by-products.

### Example 1-8: Fluorinated Zirconia Catalyst; W/F = 30.0 g·sec/cc; 350°C; 14.9 sec

The reaction was carried out in the same manner as in Example 1-1, except that the reaction was carried out at a heating temperature of 350°C for 14.9 seconds by using the fluorinated zirconia catalyst (zirconia fluorinated with hydrogen fluoride) as the catalyst. One hour after the completion of the reaction, the gas flowing out of the reaction tube was analyzed by gas chromatography, which revealed that the conversion rate was 99.5 mol%, and the selectivity of each component was as follows: 2.68 mol% for 1,1,1,4,4,4-hexafluoro-2-butyne (CF₃C≡CCF₃), 96.3 mol% for 1, 2, 3, 3, 4, 4-hexafluoro-1-cyclobutene (c-C₄F₆), 0.0127 mol% for 1, 1, 2, 3, 3, 4, 4, 4-octafluoro-1-butene (CF₂=CFCF₂CF₃) (the total amount of the E-isomer and Z-isomer), 0.118 mol% for 1,1,1,2,4,4,4-heptafluoro-2-butene (CF₃CF=CHCF₃) (the total amount of the E-isomer and Z-isomer), and 0.274 mol% for the total of other by-products.

### Example 1-9: Fluorinated Zirconia Catalyst; W/F = 15.0 g·sec/cc; 350°C; 7.5 sec

The reaction was carried out in the same manner as in Example 1-1, except that the reaction was carried out at a heating temperature of 350°C with a W/F of 15.0 g·sec/cc for 7.5 seconds by using the fluorinated zirconia catalyst (zirconia fluorinated with hydrogen fluoride) as the catalyst. One hour after the completion of the reaction, the gas flowing out of the reaction tube was analyzed by gas chromatography, which revealed that the conversion rate was 99.1 mol%, and the selectivity of each component was as follows: 3.71 mol% for 1,1,1,4,4,4-hexafluoro-2-butyne (CF₃C≡CCF₃), 95.3 mol% for 1,2,3,3,4,4-hexafluoro-1-cyclobutene (c-C₄F₆), 0.0163 mol% for 1, 1, 2, 3, 3, 4, 4, 4-octafluoro-1-butene (CF₂=CFCF₂CF₃) (the total amount of the E-isomer and Z-isomer), 0.0851 mol% for 1,1,1,2,4,4,4-heptafluoro-2-butene (CF₃CF=CHCF₃) (the total amount of the E-isomer and Z-isomer), and 0.246 mol% for the total of other by-products.

Table 1 shows the results of Examples 1-1 to 1-9.

**Table 1**

| | Example | | | | | | | | |
|---|---|---|---|---|---|---|---|---|---|
| | 1-1 | 1-2 | 1-3 | 1-4 | 1-5 | 1-6 | 1-7 | 1-8 | 1-9 |
| Catalyst | Fluorinated chromia | Fluorinated chromia | Fluorinated chromia | Fluorinated titania | Fluorinated titania | Fluorinated titania | Fluorinated zirconia | Fluorinated zirconia | Fluorinated zirconia |
| W/F | 30.0 | 30.0 | 90.0 | 30.0 | 30.0 | 30.0 | 30.0 | 30.0 | 15.0 |
| Temperature (°C) | 200 | 250 | 200 | 200 | 250 | 300 | 250 | 350 | 350 |
| Contact time (sec) | 23.1 | 23.1 | 69.3 | 32.5 | 32.5 | 32.5 | 14.9 | 14.9 | 7.5 |
| Conversion rate (mol%) | 100 | 100 | 100 | 99.0 | 99.9 | 100 | 70.1 | 99.5 | 99.1 |

| Selectivity (mol%) | | | | | | | | | |
|---|---|---|---|---|---|---|---|---|---|
| CF₃C≡CCF₃ | 99.7 | 97.2 | 99.7 | 99.3 | 23.7 | 44.5 | 94.1 | 2.68 | 3.71 |
| c-C₄F₆ | 0.162 | 2.47 | 0.118 | 0.354 | 76.0 | 55.1 | 4.18 | 96.3 | 95.3 |
| CF₂=CFCF₂CF₃ | 0.0356 | 0.0871 | 0.0254 | 0.0595 | 0.00110 | 0.00601 | 0.00100 | 0.0127 | 0.0163 |
| CF₃CF=CHCF₃ | 0.0947 | 0.262 | 0.0911 | 0.0341 | 0.00421 | 0.00200 | 0.198 | 0.118 | 0.0851 |
| Others | 0.0182 | 0.0194 | 0.0162 | 0.213 | 0.295 | 0.392 | 0.0760 | 0.274 | 0.246 |
| Total | 100 | 100 | 100 | 100 | 100 | 100 | 100 | 100 | 100 |

### Example 1-10: Fluorinated Chromia Catalyst; W/F= 15.0 g·sec/cc; 200°C; 16.2 sec

The reaction was carried out in the same manner as in Example 1-1, except that the reaction was carried out with a W/F of 15.0 g·sec/cc for 16.2 seconds. One hour after the completion of the reaction, the gas flowing out of the reaction tube was analyzed by gas chromatography, which revealed that the conversion rate was 100 mol%, and the selectivity of each component was as follows: 99.8 mol% for 1,1,1,4,4,4-hexafluoro-2-butyne (CF₃C≡CCF₃), 0.0991 mol% for 1,2,3,3,4,4-hexafluoro-1-cyclobutene (c-C₄F₆), below the detection limit (ND) for 1, 1, 2, 3, 3, 4, 4, 4-octafluoro-1-butene (CF₂=CFCF₂CF₃) (the total amount of the E-isomer and Z-isomer), 0.0850 mol% for 1,1,1,2,4,4,4-heptafluoro-2-butene (CF₃CF=CHCF₃) (the total amount of the E-isomer and Z-isomer), and 0.0159 mol% for the total of other by-products.

### Example 1-11: Chromia Catalyst; W/F= 30.0 g·sec/cc; 20°C; 25.1 sec

The reaction was carried out in the same manner as in Example 1-1, except that the reaction was carried out at a heating temperature of 20°C for 25.1 seconds by using the chromia catalyst as the catalyst. One hour after the completion of the reaction, the gas flowing out of the reaction tube was analyzed by gas chromatography, which revealed that the conversion rate was 100 mol%, and the selectivity of each component was as follows: 99.8 mol% for 1,1,1,4,4,4-hexafluoro-2-butyne (CF₃C≡CCF₃), below the detection limit (ND) for 1,2,3,3,4,4-hexafluoro-1-cyclobutene (c-C₄F₆), 0.0257 mol% for 1, 1, 2, 3, 3, 4, 4, 4-octafluoro-1-butene (CF₂=CFCF₂CF₃) (the total amount of the E-isomer and Z-isomer), 0.0511 mol% for 1,1,1,2,4,4,4-heptafluoro-2-butene (CF₃CF=CHCF₃) (the total amount of the E-isomer and Z-isomer), and 0.0832 mol% for the total of other by-products.

### Example 1-12: Chromia Catalyst; W/F= 14.0 g·sec/cc; 20°C; 11.7 sec

The reaction was carried out in the same manner as in Example 1, except that the reaction was carried out with a W/F of 14.0 g·sec/cc at a heating temperature of 20°C for 11.7 seconds by using the chromia catalyst as the catalyst. One hour after the completion of the reaction, the gas flowing out of the reaction tube was analyzed by gas chromatography, which revealed that the conversion rate was 100 mol%, and the selectivity of each component was as follows: 99.8 mol% for 1,1,1,4,4,4-hexafluoro-2-butyne (CF₃C≡CCF₃), below the detection limit (ND) for 1, 2, 3, 3, 4, 4-hexafluoro-1-cyclobutene (c-C₄F₆), 0.0187 mol% for 1, 1, 2, 3, 3, 4, 4, 4-octafluoro-1-butene (CF₂=CFCF₂CF₃) (the total amount of the E-isomer and Z-isomer), 0.0544 mol% for 1,1,1,2,4,4,4-heptafluoro-2-butene (CF₃CF=CHCF₃) (the total amount of the E-isomer and Z-isomer), and 0.0969 mol% for the total of other by-products.

### Example 1-13: Chromia Catalyst; W/F= 30.0 g·sec/cc; 50°C; 25.1 sec

The reaction was carried out in the same manner as in Example 1-1, except that the reaction was carried out at a heating temperature of 50°C for 25.1 seconds by using the chromia catalyst as the catalyst. One hour after the completion of the reaction, the gas flowing out of the reaction tube was analyzed by gas chromatography, which revealed that the conversion rate was 100 mol%, and the selectivity of each component was as follows: 99.9 mol% for 1,1,1,4,4,4-hexafluoro-2-butyne (CF₃C≡CCF₃), below the detection limit (ND) for 1,2,3,3,4,4-hexafluoro-1-cyclobutene (c-C₄F₆), 0.0201 mol% for 1, 1, 2, 3, 3, 4, 4, 4-octafluoro-1-butene (CF₂=CFCF₂CF₃) (the total amount of the E-isomer and Z-isomer), 0.0387 mol% for 1,1,1,2,4,4,4-heptafluoro-2-butene (CF₃CF=CHCF₃) (the total amount of the E-isomer and Z-isomer), and 0.0012 mol% for the total of other by-products.

### Example 1-14: Chromia catalyst; W/F= 6.0 g·sec/cc; 150°C; 5.0 sec

The reaction was carried out in the same manner as in Example 1-1, except that the reaction was carried out with a W/F of 6.0 g·sec/cc at a heating temperature of 150°C for 5.0 seconds by using the chromia catalyst as the catalyst. One hour after the completion of the reaction, the gas flowing out of the reaction tube was analyzed by gas chromatography, which revealed that the conversion rate was 100 mol%, and the selectivity of each component was as follows: 99.8 mol% for 1,1,1,4,4,4-hexafluoro-2-butyne (CF₃C≡CCF₃), 0.00311 mol% for 1,2,3,3,4,4-hexafluoro-1-cyclobutene (c-C₄F₆), 0.0274 mol% for 1,1,2,3,3,4,4,4-octafluoro-1-butene (CF₂=CFCF₂CF₃) (the total amount of the E-isomer and Z-isomer), 0.0477 mol% for 1,1,1,2,4,4,4-heptafluoro-2-butene (CF₃CF=CHCF₃) (the total amount of the E-isomer and Z-isomer), and 0.0818 mol% for the total of other by-products.

### Example 1-15: Chromia Catalyst; W/F= 30.0 g·sec/cc; 150°C; 25.1 sec

The reaction was carried out in the same manner as in Example 1-1, except that the reaction was carried out at a heating temperature of 150°C for 25.1 seconds by using the chromia catalyst as the catalyst. One hour after the completion of the reaction, the gas flowing out of the reaction tube was analyzed by gas chromatography, which revealed that the conversion rate was 100 mol%, and the selectivity of each component was as follows: 99.8 mol% for 1,1,1,4,4,4-hexafluoro-2-butyne (CF₃C≡CCF₃), 0.00154 mol% for 1,2,3,3,4,4-hexafluoro-1-cyclobutene (c-C₄F₆), 0.0272 mol% for 1,1,2,3,3,4,4,4-octafluoro-1-butene (CF₂=CFCF₂CF₃) (the total amount of the E-isomer and Z-isomer), 0.0364 mol% for 1,1,1,2,4,4,4-heptafluoro-2-butene (CF₃CF=CHCF₃) (the total amount of the E-isomer and Z-isomer), and 0.125 mol% for the total of other by-products.

### Example 1-16: Chromia Catalyst; W/F= 8.0 g·sec/cc; 200°C; 6.7 sec

The reaction was carried out in the same manner as in Example 1-1, except that the reaction was carried out with a W/F of 8.0 g·sec/cc for 6.7 seconds by using the chromia catalyst as the catalyst. One hour after the completion of the reaction, the gas flowing out of the reaction tube was analyzed by gas chromatography, which revealed that the conversion rate was 99.9 mol%, and the selectivity of each component was as follows: 99.6 mol% for 1,1,1,4,4,4-hexafluoro-2-butyne (CF₃C≡CCF₃), 0.00311 mol% for 1, 2, 3, 3, 4, 4-hexafluoro-1-cyclobutene (c-C₄F₆), below the detection limit (ND) for the 1, 1, 2, 3, 3, 4, 4, 4-octafluoro-1-butene (CF₂=CFCF₂CF₃) (the total amount of the E-isomer and Z-isomer), 0.00111 mol% for 1, 1, 1, 2, 4, 4, 4-heptafluoro-2-butene (CF₃CF=CHCF₃) (the total amount of the E-isomer and Z-isomer), and 0.416 mol% for the total of other by-products.

### Example 1-17: Chromia Catalyst; W/F= 16.0 g·sec/cc; 200°C; 13.3 sec

The reaction was carried out in the same manner as in Example 1-1, except that the reaction was carried out with a W/F of 16.0 g·sec/cc for 13.3 seconds by using the chromia catalyst as the catalyst. One hour after the completion of the reaction, the gas flowing out of the reaction tube was analyzed by gas chromatography, which revealed that the conversion rate was 100 mol%, and the selectivity of each component was as follows: 99.9 mol% for 1,1,1,4,4,4-hexafluoro-2-butyne (CF₃C≡CCF₃), 0.00311 mol% for 1, 2, 3, 3, 4, 4-hexafluoro-1-cyclobutene (c-C₄F₆), below the detection limit (ND) for 1, 1, 2, 3, 3, 4, 4, 4-octafluoro-1-butene (CF₂=CFCF₂CF₃) (the total amount of the E-isomer and Z-isomer), 0.00101 mol% for 1, 1, 1, 2, 4, 4, 4-heptafluoro-2-butene (CF₃CF=CHCF₃) (the total amount of the E-isomer and Z-isomer), and 0.0959 mol% for the total of other by-products.

### Example 1-18: Titania Catalyst; W/F= 10.0 g·sec/cc; 200°C; 11.1 sec

The reaction was carried out in the same manner as in Example 1-1, except that the reaction was carried out with a W/F of 10.0 g·sec/cc for 11.1 seconds by using the titania catalyst as the catalyst. One hour after the completion of the reaction, the gas flowing out of the reaction tube was analyzed by gas chromatography, which revealed that the conversion rate was 99.0 mol%, and the selectivity of each component was as follows: 99.4 mol% for 1,1,1,4,4,4-hexafluoro-2-butyne (CF₃C≡CCF₃), 0.254 mol% for 1, 2, 3, 3, 4, 4-hexafluoro-1-cyclobutene (c-C₄F₆), 0.059 mol% for 1, 1, 2, 3, 3, 4, 4, 4-octafluoro-1-butene (CF₂=CFCF₂CF₃) (ND; the total amount of the E-isomer and Z-isomer), 0.0341 mol% for 1,1,1,2,4,4,4-heptafluoro-2-butene (CF₃CF=CHCF₃) (the total amount of the E-isomer and Z-isomer), and 0.213 mol% for the total of other by-products.

### Example 1-19: Titania Catalyst; W/F= 14.0 g·sec/cc; 200°C; 15.5 sec

The reaction was carried out in the same manner as in Example 1-1, except that the reaction was carried out with a W/F of 14.0 g·sec/cc for 15.5 seconds by using the titania catalyst as the catalyst. One hour after the completion of the reaction, the gas flowing out of the reaction tube was analyzed by gas chromatography, which revealed that the conversion rate was 99.0 mol%, and the selectivity of each component was as follows: 99.3 mol% for 1,1,1,4,4,4-hexafluoro-2-butyne (CF₃C≡CCF₃), 0.362 mol% for 1, 2, 3, 3, 4, 4-hexafluoro-1-cyclobutene (c-C₄F₆), 0.0587 mol% for 1, 1, 2, 3, 3, 4, 4, 4-octafluoro-1-butene (CF₂=CFCF₂CF₃) (ND; the total amount of the E-isomer and Z-isomer), 0.0321 mol% for 1,1,1,2,4,4,4-heptafluoro-2-butene (CF₃CF=CHCF₃) (the total amount of the E-isomer and Z-isomer), and 0.247 mol% for the total of other by-products.

### Example 1-20: Silica-alumina Catalyst; W/F = 7.5 g·sec/cc; 20°C; 14.9 sec

The reaction was carried out in the same manner as in Example 1-1, except that the reaction was carried out with a W/F of 7.5 g·sec/cc at a heating temperature of 20°C for 14.9 seconds by using the silica-alumina catalyst as the catalyst. One hour after the completion of the reaction, the gas flowing out of the reaction tube was analyzed by gas chromatography, which revealed that the conversion rate ratio was 100 mol%, and the selectivity of each component was as follows: 99.4 mol% for 1,1,1,4,4,4-hexafluoro-2-butyne (CF₃C≡CCF₃), below the detection limit (ND) for 1, 2, 3, 3, 4, 4-hexafluoro-1-cyclobutene (c-C₄F₆), below the detection limit (ND) for 1, 1, 2, 3, 3, 4, 4, 4-octafluoro-1-butene (CF₂=CFCF₂CF₃) (the total amount of the E-isomer and Z-isomer), 0.0909 mol% for 1,1,1,2,4,4,4-heptafluoro-2-butene (CF₃CF=CHCF₃) (the total amount of the E-isomer and Z-isomer), and 0.504 mol% for the total of other by-products.

### Example 1-21: Silica-alumina Catalyst; W/F = 15.0 g·sec/cc; 20°C; 29.9 sec

The reaction was carried out in the same manner as in Example 1-1, except that the reaction was carried out with a W/F of 15.0 g·sec/cc at a heating temperature of 20°C for 29.9 seconds by using the silica-alumina catalyst as the catalyst. One hour after the completion of the reaction, the gas flowing out of the reaction tube was analyzed by gas chromatography, which revealed that the conversion rate was 100 mol%, and the selectivity of each component was as follows: 99.9 mol% for 1,1,1,4,4,4-hexafluoro-2-butyne (CF₃C≡CCF₃), below the detection limit (ND) for 1, 2, 3, 3, 4, 4-hexafluoro-1-cyclobutene (c-C₄F₆), 0.00688 mol% for 1, 1, 2, 3, 3, 4, 4, 4-octafluoro-1-butene (CF₂=CFCF₂CF₃) (the total amount of the E-isomer and Z-isomer), 0.0555 mol% for 1,1,1,2,4,4,4-heptafluoro-2-butene (CF₃CF=CHCF₃) (the total amount of the E-isomer and Z-isomer), and 0.0374 mol% for the total of other by-products.

### Example 1-22: Silica-alumina Catalyst; W/F = 30.0 g·sec/cc; 20°C; 59.8 sec

The reaction was carried out in the same manner as in Example 1-1, except that the reaction was carried out at a heating temperature of 20°C for 59.8 seconds by using the silica-alumina catalyst as the catalyst. One hour after the completion of the reaction, the gas flowing out of the reaction tube was analyzed by gas chromatography, which revealed that the conversion rate was 100 mol%, and the selectivity of each component was as follows: 99.8 mol% for 1,1,1,4,4,4-hexafluoro-2-butyne (CF₃C≡CCF₃), below the detection limit (ND) for 1, 2, 3, 3, 4, 4-hexafluoro-1-cyclobutene (c-C₄F₆), below the detection limit (ND) for 1, 1, 2, 3, 3, 4, 4, 4-octafluoro-1-butene (CF₂=CFCF₂CF₃) (the total amount of the E-isomer and Z-isomer), 0.0804 mol% for 1,1,1,2,4,4,4-heptafluoro-2-butene (CF₃CF=CHCF₃) (the total amount of the E-isomer and Z-isomer), and 0.161 mol% for the total of other by-products.

### Example 1-23: Silica-alumina Catalyst; W/F = 4.0 g·sec/cc; 100°C; 8.0 sec

The reaction was carried out in the same manner as in Example 1, except that the reaction was carried out with a W/F of 4.0 g·sec/cc at a heating temperature of 100°C for 8.0 seconds by using the silica-alumina catalyst as the catalyst. One hour after the completion of the reaction, the gas flowing out of the reaction tube was analyzed by gas chromatography, which revealed that the conversion rate was 100 mol%, and the selectivity of each component was as follows: 99.4 mol% for 1,1,1,4,4,4-hexafluoro-2-butyne (CF₃C≡CCF₃), 0.00121 mol% for 1,2,3,3,4,4-hexafluoro-1-cyclobutene (c-C₄F₆), below the detection limit (ND) for 1, 1, 2, 3, 3, 4, 4, 4-octafluoro-1-butene (CF₂=CFCF₂CF₃) (the total amount of the E-isomer and Z-isomer), 0.0909 mol% for 1,1,1,2,4,4,4-heptafluoro-2-butene (CF₃CF=CHCF₃) (the total amount of the E-isomer and Z-isomer), and 0.503 mol% for the total of other by-products.

### Example 1-24: Silica-alumina Catalyst; W/F = 4.0 g·sec/cc; 200°C; 8.0 sec

The reaction was carried out in the same manner as in Example 1-1, except that the reaction was carried out with a W/F of 4.0 g·sec/cc for 8.0 seconds by using the silica-alumina catalyst as the catalyst. One hour after the completion of the reaction, the gas flowing out of the reaction tube was analyzed by gas chromatography, which revealed that the conversion rate was 100 mol%, and the selectivity of each component was as follows: 99.5 mol% for 1,1,1,4,4,4-hexafluoro-2-butyne (CF₃C≡CCF₃), 0.0670 mol% for 1, 2, 3, 3, 4, 4-hexafluoro-1-cyclobutene (c-C₄F₆), below the detection limit (ND) for 1,1,2,3,3,4,4,4-octafluoro-1-butene (CF₂=CFCF₂CF₃) (the total amount of the E-isomer and Z-isomer), 0.0101 mol% for 1,1,1,2,4,4,4-heptafluoro-2-butene (CF₃CF=CHCF₃) (the total amount of the E-isomer and Z-isomer), and 0.467 mol% for the total of other by-products.

### Example 1-25: Silica-alumina Catalyst; W/F = 8.0 g·sec/cc; 200°C; 16.0 sec

The reaction proceeded in the same manner as in Example 1-1, except that the reaction was carried out with a W/F of 8.0 g·sec/cc for 16.0 seconds by using the silica-alumina catalyst as the catalyst. One hour after the completion of the reaction, the gas flowing out of the reaction tube was analyzed by gas chromatography, which revealed that the conversion rate was 100 mol%, and the selectivity of each component was as follows: 99.5 mol% for 1,1,1,4,4,4-hexafluoro-2-butyne (CF₃C≡CCF₃), 0.0611 mol% for 1, 2, 3, 3, 4, 4-hexafluoro-1-cyclobutene (c-C₄F₆), below the detection limit (ND) for 1, 1, 2, 3, 3, 4, 4, 4-octafluoro-1-butene (CF₂=CFCF₂CF₃) (the total amount of the E-isomer and Z-isomer), 0.00921 mol% for 1,1,1,2,4,4,4-heptafluoro-2-butene (CF₃CF=CHCF₃) (the total amount of the E-isomer and Z-isomer), and 0.480 mol% for the total of other by-products.

Table 2 shows the results of Examples 1-1 and 1-10 to 1-17, and Table 3 shows the results of Examples 1-18 to 1-25.

**Table 2**

| | Example | | | | | | | | |
|---|---|---|---|---|---|---|---|---|---|
| | 1-1 | 1-10 | 1-11 | 1-12 | 1-13 | 1-14 | 1-15 | 1-16 | 1-17 |
| Catalyst | Fluorinated chromia | Fluorinated chromia | Chromia | Chromia | Chromia | Chromia | Chromia | Chromia | Chromia |
| W/F | 30.0 | 15.0 | 30.0 | 14.0 | 30.0 | 6.0 | 30.0 | 8.0 | 16.0 |
| Temperature (°C) | 200 | 200 | 20 | 20 | 50 | 150 | 150 | 200 | 200 |
| Contact time (sec) | 23.1 | 16.2 | 25.1 | 11.7 | 25.1 | 5.0 | 25.1 | 6.7 | 13.3 |
| Conversion rate (mol%) | 100 | 100 | 100 | 100 | 100 | 100 | 100 | 99.9 | 100 |

| Selectivity (mol%) | | | | | | | | | |
|---|---|---|---|---|---|---|---|---|---|
| CF₃C≡CCF₃ | 99.7 | 99.8 | 99.8 | 99.8 | 99.9 | 99.8 | 99.8 | 99.6 | 99.9 |
| c-C₄F₆ | 0.162 | 0.0991 | ND | ND | ND | 0.00311 | 0.00154 | 0.00311 | 0.00311 |
| CF₂=CFCF₂CF₃ | 0.0356 | ND | 0.0257 | 0.0187 | 0.0201 | 0.0274 | 0.0272 | ND | ND |
| CF₃CF=CHCF₃ | 0.0947 | 0.0850 | 0.0511 | 0.0544 | 0.0387 | 0.0477 | 0.0364 | 0.00111 | 0.00101 |
| Others | 0.0182 | 0.0159 | 0.0832 | 0.0969 | 0.0012 | 0.0818 | 0.125 | 0.416 | 0.0959 |
| Total | 100 | 100 | 100 | 100 | 100 | 100 | 100 | 100 | 100 |

**Table 3**

| | Example | | | | | | | |
|---|---|---|---|---|---|---|---|---|
| | 1-18 | 1-19 | 1-20 | 1-21 | 1-22 | 1-23 | 1-24 | 1-25 |
| Catalyst | Titania | Titania | Silica-alumina | Silica-alumina | Silica-alumina | Silica-alumina | Silica-alumina | Silica-alumina |
| W/F | 10.0 | 14.0 | 7.5 | 15.0 | 30.0 | 4.0 | 4.0 | 8.0 |
| Temperature (°C) | 200 | 200 | 20 | 20 | 20 | 100 | 200 | 200 |
| Contact time (sec) | 11.1 | 15.5 | 14.9 | 29.9 | 59.8 | 8.0 | 8.0 | 16.0 |
| Conversion rate (mol%) | 99.0 | 99.0 | 100 | 100 | 100 | 100 | 100 | 100 |

| Selectivity (mol%) | | | | | | | | |
|---|---|---|---|---|---|---|---|---|
| CF₃C≡CCF₃ | 99.4 | 99.3 | 99.4 | 99.9 | 99.8 | 99.4 | 99.5 | 99.5 |
| c-C₄F₆ | 0.254 | 0.362 | ND | ND | ND | 0.00121 | 0.0670 | 0.0611 |
| CF₂=CFCF₂CF₃ | 0.059 | 0.0587 | ND | 0.00688 | ND | ND | ND | ND |
| CF₃CF=CHCF₃ | 0.0341 | 0.0321 | 0.0909 | 0.0555 | 0.0804 | 0.0909 | 0.0101 | 0.00921 |
| Others | 0.213 | 0.247 | 0.504 | 0.0374 | 0.161 | 0.503 | 0.467 | 0.480 |
| Total | 100 | 100 | 100 | 100 | 100 | 100 | 100 | 100 |

### Example 2: Requirements (C) to (F)

The following catalysts were used in Example 2 below. Fluorinated chromia catalyst (1): chromia fluorinated with hydrogen fluoride; pore volume: 0.10 mL/g; chromia (Cr₂O₃, pore volume: 0.15 mL/g) was fluorinated by allowing hydrogen fluoride to flow through under atmospheric pressure at 100 to 400°C for 6 hours.
Fluorinated chromia catalyst (2): chromia fluorinated with chlorodifluoromethane (R22); pore volume: 0.13 mL/g; chromia (Cr₂O₃, pore volume: 0.15 mL/g) was fluorinated by allowing chlorodifluoromethane (R22) to flow through under atmospheric pressure at 100 to 500°C for 6 hours.
Fluorinated silica-alumina catalyst (1): silica-alumina fluorinated with hydrogen fluoride; pore volume: 0.55 mL/g; silica-alumina (pore volume: 0.70 mL/g) was fluorinated by allowing hydrogen fluoride to flow through under atmospheric pressure at 100 to 400°C for 6 h.
Fluorinated silica-alumina catalyst (2): silica-alumina fluorinated with chlorodifluoromethane (R22); pore volume: 0.69 mL/g; silica-alumina (pore volume: 0.70 mL/g) was fluorinated by allowing chlorodifluoromethane (R22) to flow through under atmospheric pressure at 100 to 500°C for 6 hours.

All of the fluorinated alumina catalysts were obtained by fluorinating predetermined alumina by allowing hydrogen fluoride to flow through under atmospheric pressure at 100 to 400°C for 6 hours. Table 4 shows the details of the fluorinated alumina catalysts.

**Table 4**

| | Fluorinated alumina catalyst | | | | |
|---|---|---|---|---|---|
| | (1) | (2) | (3) | (4) | (5) |
| Physical properties before fluorination | | | | | |
| Specific surface area (m²/g) | 165 | 300 | 166 | 150 | 270 |
| Pore volume: (mL/g) | 0.91 | 0.61 | 0.37 | 0.51 | 0.38 |
| Physical properties after fluorination | | | | | |
| Specific surface area (m²/g) | 97.4 | 141 | 84.4 | 122 | 93.8 |
| Pore volume: (mL/g) | 1.33 | 0.43 | 0.22 | 0.39 | 0.23 |
| Composition after fluorination | | | | | |
| F (atom%) | 16.9 | 18.1 | 16.9 | 18.3 | 48.7 |

### Example 2-1: R22-fluorinated Chromia Catalyst; Pore Volume: 0.13 mL/g

As a catalyst, the fluorinated chromia catalyst (2) (chromia fluorinated with chlorodifluoromethane (R22)) was filled in a metal tubular reactor. The reaction tube was heated to 200°C, and hexafluorobutadiene (CF₂CF=CFCF₂) was fed to the reaction tube to achieve a W/F of 8 g·sec/cc to allow a reaction to proceed in a gas-phase continuous flow process. After a predetermined amount of time, the gas flowing out of the reaction tube was analyzed by gas chromatography. The results revealed that the catalyst degradation rate was -0.12%/hour. The catalyst degradation rate is represented by the slope obtained by plotting the reaction time on the horizontal axis, and the conversion rate on the vertical axis. Table 5 shows the results.

**Table 5**

| | | | | | | | | |
|---|---|---|---|---|---|---|---|---|
| Fluorinated chromia catalyst | Degradation rate: -0.12% | | | | | | | |
| Before fluorination | After fluorination | | | | | | | |
| Pore volume: 0.15 mL/g | Pore volume: 0.13 mL/g | | | | | | | |
| W/F (g·sec/cc) | 8 | 8 | 8 | 8 | 8 | 8 | 8 | 8 |
| (Reaction time) | 1 | 6 | 24 | 29 | 32 | 48 | 51 | 60 |
| Conversion rate (mol%) | 100 | 100 | 100 | 99.9 | 99.8 | 98.1 | 97.2 | 96.4 |

| Selectivity (mol%) | | | | | | | | |
|---|---|---|---|---|---|---|---|---|
| CF₃C≡CCF₃ | 99.0 | 99.4 | 99.4 | 99.6 | 99.6 | 99.6 | 99.6 | 99.7 |
| CF₂=CFCF₂CF₃ | 0.0609 | 0.0507 | 0.0582 | 0.0541 | 0.0510 | 0.0504 | 0.0441 | 0.00128 |
| c-C₄F₆ | 0.00394 | 0.000984 | 0.0442 | 0.0347 | 0.0336 | 0.0775 | 0.0822 | 0.0433 |
| CF₃CF=CHCF₃ | 0.176 | 0.198 | 0.166 | 0.106 | 0.110 | 0.0910 | 0.0923 | 0.0902 |
| C₄F₇H (except for CF₃CF=CHCF₃) | ND | ND | ND | ND | ND | ND | ND | ND |
| Others | 0.727 | 0.392 | 0.295 | 0.228 | 0.182 | 0.213 | 0.155 | 0.202 |
| Total | 100 | 100 | 100 | 100 | 100 | 100 | 100 | 100 |

### Example 2-2: R22-fluorinated Silica-Alumina Catalyst; Pore Volume: 0.69 mL/g

The reaction proceeded in the same manner as in Example 2-1, except that the fluorinated silica-alumina catalyst (2) (silica-alumina fluorinated with chlorodifluoromethane (R22)) was used as the catalyst instead of the fluorinated chromia catalyst (2). After a predetermined amount of time, the gas flowing out of the reaction tube was analyzed by gas chromatography. The results revealed that the catalyst degradation rate was -0.0014%/hour. The catalyst degradation rate is represented by the slope obtained by plotting the reaction time on the horizontal axis, and the conversion rate on the vertical axis. Table 6 shows the results.

**Table 6**

| | | | | | | | | | | |
|---|---|---|---|---|---|---|---|---|---|---|
| Fluorinated silica-alumina catalyst | | Degradation rate: -0.0014% | | | | | | | | |
| Before fluorination | | After fluorination | | | | | | | | |
| Pore volume: 0.70 mL/g | | Pore volume: 0.69 mL/g | | | | | | | | |
| W/F(g·sec/cc) | 8 | 8 | 8 | 8 | 8 | 8 | 8 | 8 | 8 | 8 |
| Reaction time (hr) | 3 | 19 | 27 | 34 | 51 | 58 | 75 | 78 | 81 | 100 |
| Conversion rate (mol%) | 100 | 100 | 100 | 100 | 100 | 99.9 | 99.9 | 99.8 | 99.8 | 99.7 |

| Selectivity (mol%) | | | | | | | | | | |
|---|---|---|---|---|---|---|---|---|---|---|
| CF₃C≡CCF₃ | 99.5 | 99.8 | 99.7 | 99.8 | 99.8 | 99.8 | 99.8 | 99.8 | 99.8 | 99.6 |
| CF₂=CFCF₂CF₃ | 0.00162 | 0.00306 | 0.00402 | 0.00509 | 0.00704 | 0.00529 | 0.00658 | 0.00595 | 0.00653 | 0.00911 |
| c-C₄F₆ | 0.0570 | 0.0793 | 0.0966 | 0.106 | 0.121 | 0.117 | 0.129 | 0.127 | 0.128 | 0.144 |
| CF₃CF=CHCF₃ | 0.104 | 0.00811 | 0.135 | 0.0986 | 0.0872 | 0.0891 | 0.0840 | 0.0816 | 0.0875 | 0.290 |
| C₄F₇H (except for CF₃CF=CHCF₃) | ND | ND | ND | ND | ND | ND | ND | ND | ND | ND |
| Others | 0.386 | 0.154 | 0.0346 | 0.0317 | 0.000 | 0.0307 | 0.000276 | 0.00203 | 0.00561 | 0.00430 |
| Total | 100 | 100 | 100 | 100 | 100 | 100 | 100 | 100 | 100 | 100 |

### Example 2-3: Fluorinated Alumina Catalyst; Pore Volume: 1.33 mL/g

The reaction proceeded in the same manner as in Example 2-1, except that the fluorinated alumina catalyst (1) was used as the catalyst instead of the fluorinated chromia catalyst (2). After a predetermined amount of time, the gas flowing out of the reaction tube was analyzed by gas chromatography. The results revealed that the catalyst degradation rate was -0.025%/hr. The catalyst degradation rate is represented by the slope obtained by plotting the reaction time on the horizontal axis, and the conversion rate on the vertical axis. Table 7 shows the results.

**Table 7**

| | | | | | |
|---|---|---|---|---|---|
| Fluorinated alumina catalyst | Degradation rate: -0.025% | | | | |
| Before fluorination | After fluorination | | | | |
| Pore volume: 0.91 mL/g | Pore volume: 1.33 mL/g | | | | |
| W/F (g·sec/cc) | 4 | 4 | 4 | 4 | 4 |
| Reaction time (hr) | 2.4 | 5.7 | 21 | 30 | 50 |
| Conversion rate (mol%) | 100 | 100 | 99.9 | 99.4 | 97.7 |

| Selectivity (mol%) | | | | | |
|---|---|---|---|---|---|
| CF₃C≡CCF₃ | 98.7 | 99.5 | 99.5 | 99.5 | 99.4 |
| CF₂=CFCF₂CF₃ | ND | ND | ND | ND | ND |
| c-C₄F₆ | 0.273 | 0.300 | 0.327 | 0.302 | 0.358 |
| CF₃CF=CHCF₃ | 0.295 | 0.169 | 0.110 | 0.192 | 0.175 |
| C₄F₇H (except for CF₃CF=CHCF₃) | ND | ND | ND | ND | ND |
| Others | 0.703 | 0.0755 | 0.0697 | 0.0465 | 0.063 |
| Total | 100 | 100 | 100 | 100 | 100 |

### Example 2-4: Fluorinated Alumina Catalyst; Pore Volume: 0.43 mL/g

The reaction proceeded in the same manner as in Example 2-1, except that fluorinated alumina catalyst (2) was used as the catalyst instead of fluorinated chromia catalyst (2). After a predetermined amount of time, the gas flowing out of the reaction tube was analyzed by gas chromatography. The results revealed that the catalyst degradation rate was -0.026%/hr. The catalyst degradation rate is represented by the slope obtained by plotting the reaction time on the horizontal axis, and the conversion rate on the vertical axis. Table 8 shows the results.

**Table 8**

| | | | | | |
|---|---|---|---|---|---|
| Fluorinated alumina catalyst | Degradation rate: -0.026% | | | | |
| Before fluorination | After fluorination | | | | |
| Pore volume: 0.61 mL/g | Pore volume: 0.43 mL/g | | | | |
| W/F (g·sec/cc) | 4 | 4 | 4 | 4 | 4 |
| Reaction time (hr) | 1.5 | 5.0 | 20 | 27 | 45 |
| Conversion rate (mol%) | 99.8 | 99.8 | 99.6 | 99.3 | 97.2 |

| Selectivity (mol%) | | | | | |
|---|---|---|---|---|---|
| CF₃C≡CCF₃ | 99.6 | 99.6 | 99.6 | 99.6 | 99.3 |
| CF₂=CFCF₂CF₃ | 0.00104 | 0.00229 | 0.00416 | 0.00400 | 0.00674 |
| c-C₄F₆ | 0.199 | 0.219 | 0.244 | 0.257 | 0.271 |
| CF₃CF=CHCF₃ | 0.128 | 0.108 | 0.0927 | 0.112 | 0.180 |
| C₄F₇H (except for CF₃CF=CHCF₃) | ND | ND | ND | ND | ND |
| Others | 0.0823 | 0.0335 | 0.0400 | 0.0468 | 0.255 |
| Total | 100 | 100 | 100 | 100 | 100 |

### Comparative Example 2-1: Fluorinated Alumina Catalyst; Pore

### Volume: 0.22 mL/g

The reaction proceeded in the same manner as in Example 2-1, except that the fluorinated alumina catalyst (3) was used as the catalyst instead of the fluorinated chromia catalyst (2). After a predetermined amount of time, the gas flowing out of the reaction tube was analyzed by gas chromatography. The results revealed that the catalyst degradation rate was -0.65%/hr. The catalyst degradation rate is represented by the slope obtained by plotting the reaction time on the horizontal axis, and the conversion rate on the vertical axis. Table 9 shows the results.

**Table 9**

| | | | |
|---|---|---|---|
| Fluorinated alumina catalyst | Degradation rate: -0.65% | | |
| Before fluorination | After fluorination | | |
| Pore volume: 0.37 mL/g | Pore volume: 0.22 mL/g | | |
| W/F (g·sec/cc) | 4 | 4 | 4 |
| Reaction (hr) | 2.4 | 5.8 | 10 |
| Conversion rate (mol%) | 99.5 | 97.2 | 94.3 |

| Selectivity (mol%) | | | |
|---|---|---|---|
| CF₃C≡CCF₃ | 99.5 | 99.3 | 99.6 |
| CF₂=CFCF₂CF₃ | ND | ND | ND |
| c-C₄F₆ | 0.214 | 0.230 | 0.0247 |
| CF₃CF=CHCF₃ | 0.160 | 0.410 | 0.116 |
| C₄F₇H (except for CF₃CF=CHCF₃) | ND | ND | ND |
| Others | 0.104 | 0.0496 | 0.263 |
| Total | 100 | 100 | 100 |

### Example 2-5: Fluorinated Alumina Catalyst; Pore Volume: 0.39 mL/g

The reaction proceeded in the same manner as in Example 2-1, except that the fluorinated alumina catalyst (4) was used as the catalyst instead of the fluorinated chromia catalyst (2). After a predetermined amount of time, the gas flowing out of the reaction tube was analyzed by gas chromatography. The results revealed that the catalyst degradation rate was -0.29%/hour. The catalyst degradation rate is represented by the slope obtained by plotting the reaction time on the horizontal axis, and the conversion rate on the vertical axis. Table 10 shows the results.

**Table 10**

| | | | |
|---|---|---|---|
| Fluorinated alumina catalyst | Degradation rate: -0.29% | | |
| Before fluorination | After fluorination | | |
| Pore volume: 0.51 mL/g | Pore volume: 0.39 mL/g | | |
| W/F (g·sec/cc) | 4 | 4 | 4 |
| Reaction time (hr) | 10 | 28 | 47 |
| Conversion rate (mol%) | 100 | 94.3 | 88.2 |

| Selectivity (mol%) | | | |
|---|---|---|---|
| CF₃C≡CCF₃ | 99.8 | 99.7 | 99.6 |
| CF₂=CFCF₂CF₃ | 0.00688 | 0.00612 | 0.00987 |
| c-C₄F₆ | 0.198 | 0.217 | 0.241 |
| CF₃CF=CHCF₃ | 0.00702 | 0.104 | 0.097 |
| C₄F₇H (except for CF₃CF=CHCF₃) | 0.00806 | ND | ND |
| Others | 0.00123 | 0.000386 | 0.00942 |
| Total | 100 | 100 | 100 |

### Comparative Example 2-2: Fluorinated Alumina Catalyst; Pore

### Volume: 0.23 mL/g

The reaction proceeded in the same manner as in Example 2-1, except that the fluorinated alumina catalyst (5) was used as the catalyst instead of the fluorinated chromia catalyst (2). After a predetermined amount of time, the gas flowing out of the reaction tube was analyzed by gas chromatography. The results revealed that the catalyst degradation rate was -0.67%/hr. The catalyst degradation rate is represented by the slope obtained by plotting the reaction time on the horizontal axis, and the conversion rate on the vertical axis. Table 11 shows the results.

**Table 11**

| | | | |
|---|---|---|---|
| Fluorinated alumina catalyst | Degradation rate: -0.67% | | |
| Before fluorination | After fluorination | | |
| Pore volume: 0.38 mL/g | Pore volume: 0.23 mL/g | | |
| W/F (g·sec/cc) | 4 | 4 | 4 |
| Reaction time (hr) | 1.0 | 15 | 20 |
| Conversion rate (mol%) | 99.7 | 93.3 | 83.9 |

| Selectivity (mol%) | | | |
|---|---|---|---|
| CF₃C≡CCF₃ | 99.6 | 99.5 | 99.1 |
| CF₂=CFCF₂CF₃ | 0.00303 | 0.00488 | 0.00426 |
| c-C₄F₆ | 0.142 | 0.234 | 0.302 |
| CF₃CF=CHCF₃ | 0.115 | 0.218 | 0.127 |
| C₄F₇H (except for CF₃CF=CHCF₃) | ND | ND | ND |
| Others | 0.132 | 0.0511 | 0.437 |
| Total | 100 | 100 | 100 |

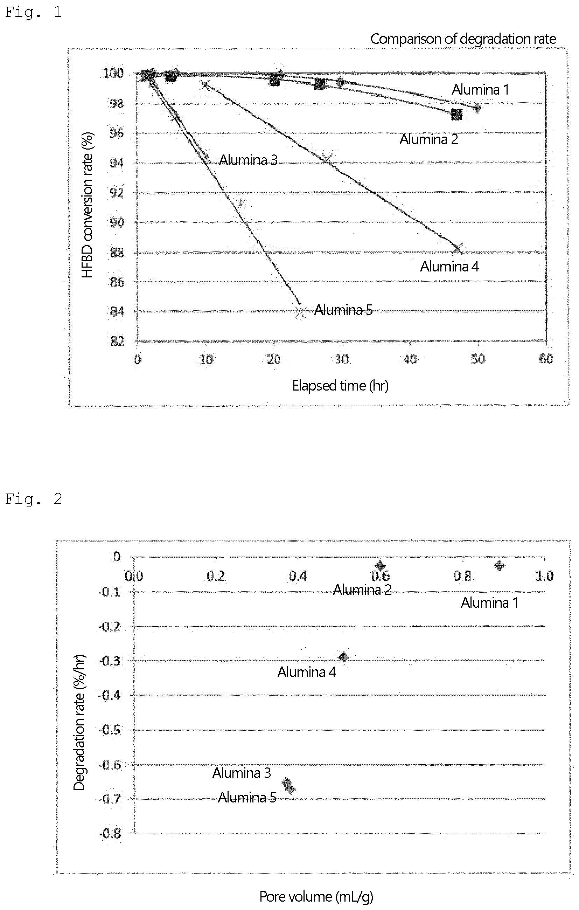

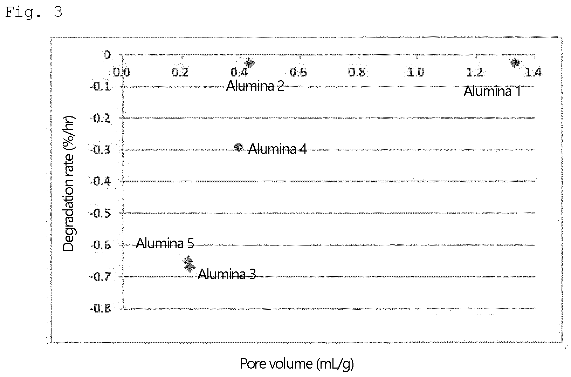

Based on the results shown in Tables 7 to 11 above, Fig. 1 shows the relationship between the elapsed time (contact time) and the conversion rate, Fig. 2 shows the relationship between the pore volume before fluorination and the degradation rate, and Fig. 3 shows the relationship between the pore volume after fluorination and the degradation rate.

### Example 3: Requirement (G)

In Example 3 below, a chromia catalyst (Cr₂O₃) or an alumina catalyst (Al₂O₃) was used as a catalyst.

The water content in hexafluorobutadiene was adjusted by adding water or by performing dehydration with molecular sieves, to achieve a predetermined water content. The water content was measured by the Karl Fischer method.

### Example 3-1: Chromia Catalyst; Water Content: 20 ppm

A metal tubular reactor was filled with nitrogen gas to create a nitrogen gas atmosphere, and then chromia catalyst was fed as a catalyst. The reaction tube was heated to 200°C, and hexafluorobutadiene (CF₂CF=CFCF₂) was fed to the reaction tube to achieve a W/F of 8 g·sec/cc to allow a reaction to proceed in a gas-phase continuous flow process. The water content in the hexafluorobutadiene at the start of the reaction was 20 ppm by mass, and the water content in the reaction system from the start to the end of the reaction was adjusted to 20 ppm. After a predetermined amount of time, the gas flowing out of the reaction tube was analyzed by gas chromatography. The results revealed that the catalyst degradation rate was -0.12%/hour. The catalyst degradation rate is represented by the slope obtained by plotting the reaction time on the horizontal axis, and the conversion rate on the vertical axis. Table 12 shows the results.

**Table 12**

| Chromia catalyst | | | | | | | | |
|---|---|---|---|---|---|---|---|---|
| Water content: 20 ppm | | | | Degradation rate: -0.12% | | | | |
| Reaction time (hr) | 2.21 | 7.21 | 21.8 | 27.0 | 48.4 | 52.5 | 60.1 | 67.0 |
| Conversion rate (mol%) | 100 | 100 | 100 | 99.9 | 99.8 | 98.1 | 97.1 | 96.4 |

| Selectivity (mol%) | | | | | | | | |
|---|---|---|---|---|---|---|---|---|
| CF₃C≡CCF₃ | 99.0 | 99.3 | 99.4 | 99.6 | 99.6 | 99.6 | 99.6 | 99.7 |
| CF₂=CFCF₂CF₃ | 0.0609 | 0.0507 | 0.0582 | 0.0541 | 0.0510 | 0.0504 | 0.0441 | 0.00128 |
| c-C₄F₆ | 0.00394 | 0.000984 | 0.0442 | 0.0347 | 0.0336 | 0.0775 | 0.0822 | 0.0433 |
| CF₃CF=CHCF₃ | 0.176 | 0.198 | 0.166 | 0.106 | 0.110 | 0.0910 | 0.0923 | 0.0902 |
| C₄F₇H (except for CF₃CF=CHCF₃) | ND | ND | ND | ND | ND | ND | ND | ND |
| Others | 0.727 | 0.492 | 0.295 | 0.228 | 0.182 | 0.213 | 0.155 | 0.192 |
| Total | 100 | 100 | 100 | 100 | 100 | 100 | 100 | 100 |

### Example 3-2: Chromia Catalyst; Water Content: 2 ppm

The reaction proceeded in the same manner as in Example 3-1, except that the water content in the reaction system from the start to the end of the reaction was adjusted to 2 ppm by using hexafluorobutadiene with a water content of 2 ppm by mass at the start of the reaction instead of hexafluorobutadiene with a water content of 20 ppm by mass at the start of the reaction. After a predetermined amount of time, the gas flowing out of the reaction tube was analyzed by gas chromatography. The results revealed that the catalyst degradation rate was -0.12%/hour. The catalyst degradation rate is represented by the slope obtained by plotting the reaction time on the horizontal axis, and the conversion rate on the vertical axis. Table 13 shows the results.

**Table 13**

| Chromia catalyst | | | | | | | | |
|---|---|---|---|---|---|---|---|---|
| Water content: 2 ppm | | | | Degradation rate: -0.12% | | | | |
| Reaction time (hr) | 1.21 | 6.42 | 24.4 | 29.0 | 31.5 | 48.2 | 51.4 | 60.0 |
| Conversion rate (mol%) | 100 | 100 | 100 | 99.9 | 99.8 | 98.1 | 97.2 | 96.4 |

| Selectivity (mol%) | | | | | | | | |
|---|---|---|---|---|---|---|---|---|
| CF₃C≡CCF₃ | 99.0 | 99.4 | 99.5 | 99.6 | 99.6 | 99.6 | 99.6 | 99.7 |
| CF₂=CFCF₂CF₃ | 0.0609 | 0.0507 | 0.0582 | 0.0541 | 0.0510 | 0.0504 | 0.0441 | 0.0013 |
| c-C₄F₆ | 0.00394 | 0.000984 | 0.0442 | 0.0347 | 0.0336 | 0.0775 | 0.0822 | 0.0433 |
| CF₃CF=CHCF₃ | 0.176 | 0.198 | 0.166 | 0.106 | 0.110 | 0.0910 | 0.0923 | 0.0902 |
| C₄F₇H (except for CF₃CF=CHCF₃) | ND | ND | ND | ND | ND | ND | ND | ND |
| Others | 0.727 | 0.392 | 0.275 | 0.228 | 0.182 | 0.213 | 0.155 | 0.202 |
| Total | 100 | 100 | 100 | 100 | 100 | 100 | 100 | 100 |

### Example 3-3: Alumina catalyst; Water Content: 20 ppm

The reaction proceeded in the same manner as in Example 3-1, except that the alumina catalyst was used instead of the chromia catalyst. After a predetermined amount of time, the gas flowing out of the reaction tube was analyzed by gas chromatography. The results revealed that the catalyst degradation rate was -0.0302%/hr. The catalyst degradation rate is represented by the slope obtained by plotting the reaction time on the horizontal axis, and the conversion rate on the vertical axis. Table 14 shows the results.

**Table 14**

| Alumina catalyst | | | |
|---|---|---|---|
| Water content: 20 ppm | Degradation rate: -0.0302% | | |
| Reaction time (hr) | 10.2 | 16.3 | 34.1 |
| Conversion rate (mol%) | 100 | 99.7 | 99.2 |

| Selectivity (mol%) | | | |
|---|---|---|---|
| CF₃C≡CCF₃ | 99.6 | 99.6 | 99.7 |
| CF₂=CFCF₂CF₃ | ND | 0.00101 | 0.00611 |
| c-C₄F₆ | 0.204 | 0.215 | 0.219 |
| CF₃CF=CHCF₃ | 0.161 | 0.129 | 0.103 |
| C₄F₇H (except for CF₃CF=CHCF₃) | 0.0120 | ND | ND |
| Others | 0.00540 | 0.0163 | 0.000435 |
| Total | 100 | 100 | 100 |

### Examples 3-4: Alumina Catalyst; Water Content: 2 ppm

The reaction proceeded in the same manner as in Example 3-1, except that the alumina catalyst was used instead of the chromia catalyst, and that the water content in the reaction system from the start to the end of the reaction was adjusted to 2 ppm by using hexafluorobutadiene with a water content of 2 ppm by mass at the start of the reaction instead of hexafluorobutadiene with a water content of 20 ppm by mass at the start of the reaction. After a predetermined amount of time, the gas flowing out of the reaction tube was analyzed by gas chromatography. The results revealed that the catalyst degradation rate was -0.08%/hr. The catalyst degradation rate is represented by the slope obtained by plotting the reaction time on the horizontal axis, and the conversion rate on the vertical axis. Table 15 shows the results.

**Table 15**

| Alumina catalyst | | | |
|---|---|---|---|
| Water content: 2 ppm | Degradation rate: -0.08% | | |
| Reaction time (hr) | 9.02 | 15.2 | 32.3 |
| Conversion rate (mol%) | 100 | 99.7 | 99.2 |

| Selectivity (mol%) | | | |
|---|---|---|---|
| CF₃C≡CCF₃ | 99.6 | 99.6 | 99.7 |
| CF₂=CFCF₂CF₃ | ND | 0.00009 | 0.0070 |
| c-C₄F₆ | 0.204 | 0.215 | 0.219 |
| CF₃CF=CHCF₃ | 0.161 | 0.129 | 0.100 |
| C₄F₇H (except for CF₃CF=CHCF₃) | 0.0120 | ND | ND |
| Others | 0.00540 | 0.0173 | 0.00254 |
| Total | 100 | 100 | 100 |

## Claims

1. A method for producing a perfluoroalkyne compound, the method comprising reacting a perfluoroalkadiene compound in the presence of a catalyst to obtain a perfluoroalkyne compound, and satisfying one of the following (A) to (G):
(A) the catalyst comprises at least one catalyst selected from the group consisting of catalysts containing a transition metal element, and catalysts containing at least two elements that belong to Groups 3 to 14 of the periodic table of elements;
(B) the catalyst comprises a catalyst containing at least one element that belongs to Groups 3 to 14 of the periodic table, and the contact time between the catalyst and the perfluoroalkadiene compound represented by formula (2) is 30 seconds or less;
(C) the catalyst comprises at least one catalyst selected from the group consisting of fluorinated chromium oxide having a pore volume of 0.08 mL/g or more, fluorinated alumina having a pore volume of 0.35 mL/g or more, and fluorinated silica alumina having a pore volume of 0.50 mL/g or more;
(D) the catalyst comprises a fluorinated metal oxide having a pore volume of 0.35 mL/g or more;
(E) the catalyst comprises a metal oxide fluorinated by reacting a metal oxide with at least one compound selected from the group consisting of hydrofluorocarbon, hydrochlorofluorocarbon, and chlorofluorocarbon;
(F) the catalyst comprises one or more catalysts obtained by fluorinating at least one metal oxide selected from the group consisting of chromium oxide having a pore volume of 0.10 mL/g or more, alumina having a pore volume of 0.45 mL/g or more, and silica alumina having a pore volume of 0.50 mL/g or more; and
(G) at least a part of the reaction of the perfluoroalkadiene compound from the start to the end of the reaction is performed under conditions in which the water content of the reaction system is 30 ppm by mass or less, based on the mass of the perfluoroalkadiene compound defined as 100 mass%.

2. The production method according to claim 1, wherein the perfluoroalkyne compound is represented by formula (1):
CR¹₂R²-C≡C-CR³R⁴₂ (1)
wherein R¹ to R⁴ are the same or different and represent a fluorine atom or a perfluoroalkyl group.

3. The production method according to claim 1 or 2, wherein the perfluoroalkadiene compound is represented by formula (2) :
CR¹₂=CR²-CR³=CR⁴₂ (2)
wherein R¹ to R⁴ are the same or different and represent a fluorine atom or a perfluoroalkyl group.

4. The production method according to any one of claims 1 to 3, wherein the method satisfies the (A) or (B), and the catalyst is at least one catalyst selected from the group consisting of:
catalysts containing at least one transition metal element that belongs to Groups 4 to 6 of the periodic table; and
catalysts containing at least two elements that belong to Groups 4 to 6 and Groups 13 to 14 of the periodic table.

5. The production method according to any one of claims 1 to 4, wherein the method satisfies the (A) or (B), and the catalyst is at least one catalyst selected from the group consisting of optionally fluorinated chromium oxide catalysts, optionally fluorinated titanium oxide catalysts, optionally fluorinated zirconia catalysts, and optionally fluorinated silica-alumina catalysts.

6. The production method according to any one of claims 1 to 3, wherein the method satisfies the (D) and the metal of the fluorinated metal oxide includes at least one element that belongs to Groups 3 to 14 of the periodic table.

7. The production method according to any one of claims 1 to 3, wherein the method satisfies the (E) and the metal oxide before fluorination has a pore volume of 0.45 mL/g or more.

8. The production method according to claim 1, 2, 3, or 7, wherein the method satisfies the (E) and the metal of the metal oxide before fluorination includes at least one element that belongs to Groups 3 to 14 of the periodic table.

9. The production method according to any one of claims 1 to 3, wherein the method satisfies the (G) and the catalyst contains at least one element that belongs to Groups 3 to 14 of the periodic table.

10. The production method according to any one of claims 1 to 9, wherein the reaction of the perfluoroalkadiene compound is performed in a gas phase.

11. The production method according to any one of claims 1 to 10, wherein the reaction of the perfluoroalkadiene compound is performed at 170°C or more.

12. The production method according to any one of claims 1 to 11, wherein the reaction of the perfluoroalkadiene compound produces a perfluorocycloalkene compound in addition to the perfluoroalkyne compound.

13. The production method according to claim 12, wherein the perfluorocycloalkene compound is represented by formula (3): 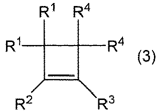 wherein R¹ to R⁴ are as defined above.

14. A method for producing a perfluoroalkyne compound, the method comprising obtaining the perfluoroalkadiene compound using as a substrate the perfluorocycloalkene compound obtained as a by-product by the production method of claim 12 or 13.

15. The production method according to claim 14, wherein the perfluoroalkyne compound is represented by formula (1):
CR¹₂R²-C≡C-CR³R⁴₂ (1)
wherein R¹ to R⁴ are the same or different and represent a fluorine atom or a perfluoroalkyl group.

16. A composition comprising a perfluoroalkyne compound and a perfluorocycloalkene compound,
the perfluoroalkyne compound being represented by formula (1):
CR¹₂R²-C≡C-CR³R⁴₂ (1)
wherein R¹ to R⁴ are the same or different and represent a fluorine atom or a perfluoroalkyl group,
the perfluorocycloalkene compound being represented by formula (3) : 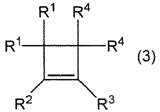 wherein R¹ to R⁴ are as defined above, and
the composition containing the perfluoroalkyne compound represented by formula (1) in an amount of 40 to 99.999 mol%, based on the total amount of the composition defined as 100 mol%.

17. The composition according to claim 16, which is for use as an etching gas or a building block for organic synthesis.

18. A catalyst for use in reacting a perfluoroalkadiene compound to obtain a perfluoroalkyne compound,
the catalyst satisfying the following (C) or (D):
(C) the catalyst contains at least one member selected from the group consisting of fluorinated chromium oxide having a pore volume of 0.08 mL/g or more, fluorinated alumina having a pore volume of 0.35 mL/g or more, and fluorinated silica alumina having a pore volume of 0.50 mL/g or more;
(D) the catalyst contains a fluorinated metal oxide having a pore volume of 0.35 mL/g or more.

19. The catalyst according to claim 18, wherein the catalyst satisfies the (D) and the metal of the fluorinated metal oxide includes at least one element that belongs to Groups 3 to 14 of the periodic table.

20. A method for producing a catalyst for use in reacting a perfluoroalkadiene compound to obtain a perfluoroalkyne compound, the method comprising the following step (E) or (F):
(E) reacting a metal oxide with at least one compound selected from the group consisting of hydrofluorocarbon, hydrochlorofluorocarbon, and chlorofluorocarbon to thereby fluorinate the metal oxide;
(F) fluorinating at least one metal oxide selected from the group consisting of chromium oxide having a pore volume of 0.10 mL/g or more, alumina having a pore volume of 0.45 mL/g or more, and silica-alumina having a pore volume of 0.50 mL/g or more.

21. The production method according to claim 20, wherein the method satisfies the (E) and the metal oxide before fluorination has a pore volume of 0.45 mL/g or more.

22. The production method according to claim 20 or 21, wherein the method satisfies the (E) and the metal of the metal oxide before fluorination includes at least one element that belongs to Groups 3 to 14 of the periodic table.
